(19) Europäisches Patentamt / European Patent Office / Office européen des brevets

(11) **EP 2 650 413 B1**

(12) **EUROPÄISCHE PATENTSCHRIFT**

(45) Veröffentlichungstag und Bekanntmachung des Hinweises auf die Patenterteilung:
**01.05.2019 Patentblatt 2019/18**

(21) Anmeldenummer: **13001246.1**

(22) Anmeldetag: **12.03.2013**

(51) Int Cl.:
*D01D 10/02* *(2006.01)*   *D01F 6/14* *(2006.01)*
*D01F 6/50* *(2006.01)*   *A61L 27/16* *(2006.01)*
*A61L 27/52* *(2006.01)*   *A61L 27/56* *(2006.01)*
*A61L 15/22* *(2006.01)*   *A61L 15/24* *(2006.01)*
*A61L 15/42* *(2006.01)*   *A61L 17/10* *(2006.01)*
*A61L 26/00* *(2006.01)*

(54) **HYDROGELIERENDE FASERGEBILDE**

HYDROGELLING FIBRES AND FIBRE STRUCTURES

FIBRES FORMANT UN HYDROGEL ET FORMATION FIBREUSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.04.2012 DE 102012007307**

(43) Veröffentlichungstag der Anmeldung:
**16.10.2013 Patentblatt 2013/42**

(73) Patentinhaber: **Carl Freudenberg KG**
**69469 Weinheim (DE)**

(72) Erfinder:
• **Schmitz, Wiebke**
**81373 München (DE)**
• **Schlesselmann, Bernd**
**69469 Weinheim (DE)**
• **Krampfl, Katharina**
**74613 Öhringen (DE)**
• **Pehr, Marc**
**69514 Laudenbach (DE)**

(56) Entgegenhaltungen:
EP-A2- 0 745 708   WO-A1-2009/085679
WO-A1-2012/048768

• JIANQI F ET AL: "PVA/PAA thermo-crosslinking hydrogel fiber: preparation and pH-sensitive properties in electrolyte solution", EUROPEAN POLYMER JOURNAL, PERGAMON PRESS LTD. OXFORD, GB, Bd. 38, Nr. 8, 1. August 2002 (2002-08-01) , Seiten 1653-1658, XP004354769, ISSN: 0014-3057, DOI: 10.1016/S0014-3057(02)00032-0
• LEI LI ET AL: "Ultra-fine polyelectrolyte hydrogel fibres from poly(acrylic acid)/poly(vinyl alcohol); Ultra-fine polyelectrolyte hydrogel fibres from poly(acrylic acid)/poly(vinyl alcohol)", NANOTECHNOLOGY, IOP, BRISTOL, GB, Bd. 16, Nr. 12, 1. Dezember 2005 (2005-12-01), Seiten 2852-2860, XP020090875, ISSN: 0957-4484, DOI: 10.1088/0957-4484/16/12/020
• AHMET ÇAY ET AL: "Properties of electrospun poly(vinyl alcohol) hydrogel nanofibers crosslinked with 1,2,3,4-butanetetracarboxylic acid", JOURNAL OF APPLIED POLYMER SCIENCE, Bd. 129, Nr. 6, 18. Februar 2013 (2013-02-18), Seiten 3140-3149, XP055073782, ISSN: 0021-8995, DOI: 10.1002/app.39036

**Beschreibung**

Technisches Gebiet

Die vorliegende Erfindung betrifft hydrogelierende zwei- oder dreidimensionale Fasergebilde, hergestellt aus einem ersten Faserrohmaterial, wobei das erste Faserrohmaterial wasserlöslichen Polyvinylalkohol und/oder Polyvinylalkohol-Copolymer enthält, sowie ein dazugehöriges Herstellungsverfahren. Des Weiteren betrifft die Erfindung die Verwendung von derartigen Fasergebilden zur Wundversorgung, insbesondere in Produkten zur medizinischen Versorgung, wie Wundauflagen, sowie in Hygiene- und Kosmetikprodukten oder dergleichen. Die Erfindung betrifft ferner Produkte zur medizinischen Versorgung, insbesondere Wundauflagen, sowie Hygiene- und Kosmetikprodukten.

Die erfindungsgemäßen Fasergebilde können vorteilhafter Weise im direkten Kontakt zur Wunde oder zum Körper verwendet werden. Aus den erfindungsgemäßen Fasergebilden hergestellte Produkte für die Wundversorgung quellen mit wässrigen Lösungen oder Wundexsudat und bilden ein stabiles Hydrogel aus, welches eine außerordentlich hohe Höchstzugkraft und Höchstzugkraftdehnung besitzt. Dadurch lassen sich Wundauflagen, die die erfindungsgemäßen Fasergebilde enthalten, in einem Stück wieder von der Wunde entfernen. Zudem besitzen die erfindungsgemäßen Fasergebilde eine besonders hohe Aufnahmekapazität sowie ein besonders hohes Rückhaltevermögen (Retention) für wässrige Lösungen.

Stand der Technik

Aus der WO 01/30407 A1 ist ein Verfahren zur Herstellung von Hydrogelen zur Verwendung als Wundauflagen bekannt, mit dem Verbrennungen oder andere Hautverletzungen behandelt werden können. Im Zuge des Verfahrens wird eine wässrige Lösung von Polyvinylalkohol, Agar-Agar und zumindest eines weiteren natürlichen Polymers zubereitet. Diese Lösung wird bei 70-80°C in Einweg-Plastikgefäßen abgefüllt und versiegelt. Nach Abkühlung auf Raumtemperatur werden die in den Einweg-Plastikgefäßen abgefüllten Proben bestrahlt und damit sterilisiert.

In der WO 2005/103097 A1 sind Hydrogele beschrieben, die zumindest ein Polyvinylalkohol-Sternpolymer aufweisen. Dabei werden die Hydrogele durch wiederholtes Einfrieren und Auftauen einer wässrigen Lösung enthaltend zumindest ein Polyvinylalkohol- Sternpolymer und optional weitere Komponenten hergestellt. Des Weiteren können derartige Hydrogele durch Einwirkung ionisierender Strahlung auf eine wässrige Lösung enthaltend zumindest ein Polyvinylalkohol - Sternpolymer oder durch eine Reaktion eines Polyvinylalkohol - Sternpolymers in wässriger Lösung mit vernetzenden Reagenzien hergestellt werden.

Nachteilig an den derzeit bekannten Methoden zur Herstellung von Hydrogelen, insbesondere für die Wundbehandlung, ist die aufwändige Herstellungsweise und die problematische Weiterverarbeitung der Hydrogele, sowie gegebenenfalls das Auftreten von chemischen Verunreinigungen in den zum Beispiel durch eine chemische Reaktion vernetzten Hydrogelen. Zudem besitzen Hydrogel-Filme im Gegensatz zu Fasern und Fasergebilden eine kleinere Oberfläche, wodurch sie eine geringere Absorptionskapazität für Wasser oder wässrige Lösungen aufweisen. Besonders bei der Verwendung von Polyvinylalkohol als Rohmaterial für Hydrogele ist darauf zu achten, dass der Polyvinylalkohol einen hohen Vernetzungsgrad aufweist, da sich sonst keine Hydrogele, sondern Lösungen des Polyvinylalkohols in dem flüssigen Medium ausbilden. Wünschenswert ist demzufolge eine hohe Stabilität des Polyvinylalkohols gegenüber Wasser oder wässrigen Lösungen. Außerdem zeichnen sich gerade Polyvinylalkohol und Polyvinylalkohol-Copolymere durch eine hohe Biokompatibilität und Bioverträglichkeit aus, so dass ein zunehmender Bedarf an weiteren Ausführungsformen von Hydrogelen oder hydrogelierenden Materialien mit Polyvinylalkohol und/oder PolyvinylalkoholCopolymeren besteht, die zudem kostengünstig und einfach herzustellen sind und eine unproblematische Weiterverarbeitung ermöglichen.

In J Mater Sci (2010) 45:2456-2465 wird ein Verfahren zur Herstellung von Nanofasern und Fasergebilden aus Polyvinylalkohol mittels Elektrospinnen beschrieben, in dem die Fasern bzw. Fasergebilde mittels einer Temperaturbehandlung gegenüber wässrigen Lösungen stabilisiert werden. Nachteilig an Fasergebilden aus Nanofasern ist, dass diese aufgrund ihres Faserdurchmessers zwischen 244 bis 270nm eine sehr geringe Festigkeit und Höchstzugkraftdehnung sowie eine nur geringe Absorptionskapazität zeigen. Zudem sind die beschriebenen Fasern gegenüber wässrigen Lösungen stabilisiert, so dass sie keine gelierenden Eigenschaften besitzen, nicht in wässriger Lösung quellen sowie nicht geeignet sind Wasser in der Faser einzuschließen (fehlendes Rückhaltevermögen).

Wundauflagen aus hydrogelierenden Fasern z.B. aus Carboxymethylcellulose oder modifizierter Cellulose sind grundsätzlich bekannt. Diese bilden jedoch ein sehr weiches Hydrogel mit geringer Höchstzugkraft und Höchstzugkraftdehnung mit der Wundflüssigkeit aus. Nachteilig hieran ist, dass sie schlecht in einem Stück von der Wunde oder aus der Wundhöhle wieder entfernt werden können. So kann es passieren dass Reste der Wundauflage in der Wunde verbleiben, die aufwändig durch Reinigung der Wunde wieder entfernt werden müssen. Dies bedeutet einen erhöhten Zeit- und damit auch Kostenaufwand für das Krankenhauspersonal. Zudem kann die Wunde durch die Reinigung wieder verletzt oder geschädigt werden.

Fasern aus Polyvinylalkohol sind kommerziell in verschiedenen Typen erhältlich und umfassen Polyvinylalkohol unterschiedlicher Wasserlöslichkeit. Wasserunlösliche Typen der Polyvinylalkohole sind beispielsweise die sogenannten High Strength-Polyvinylalkohol-Fasern mit einer besonders hohen Höchstzugkraft im trockenen Zustand. Handelsübliche wasserlösliche Fasern aus Polyvinylalkohol sind mit einer temperaturabhängigen Wasserlöslichkeit z.B. einer Wasserlöslichkeit oberhalb einer Temperatur von 90°C, von 70°C, von 60°C, von 40°C oder 20°C erhältlich. Handelsübliche Fasern aus Polyvinylalkohol können zwar in ihrer Wasserlöslichkeit variieren, sie weisen aber keine hydrogelierenden Eigenschaften auf und zeigen somit auch kein Rückhaltevermögen für Wasser.

Jianqi F et al., EUROPEAN POLYMER JOUR, 20020801 PERGAMON PRESS LTD. OXFORD, GB, XP004354769, Vol:38, Nr:8, S. 1653 - 1658 beschreibt die Herstellung von thermovernetzten Hydrogelfasern, bestehend aus Poly(vinylalkohol) (PVA) und Poly(acrylsäure) (PAA). Es werden keine zwei-, oder dreidimensionalen Fasergebilde beschrieben.

WO 2009/085679 beschreibt Verbundvliesstoffe, die einen Anteil an Fasern mit einem mittleren Faserdurchmesser von weniger als 1 $\mu$m haben und einen Anteil an Fasern mit einem mittleren Durchmesser von mindestens 1 $\mu$m.

EP 0 745 708 A1 beschreibt Fasern auf Basis von Polyvinylalkohol mit einem spezifischen Elastizitätsmodul und Heißwasserschrumpf. Es werden ebenfalls keine zwei-, oder dreidimensionalen Fasergebilde beschrieben.

Lei Li et al., NANOTECHNOLOGY, 20051201 IOP, BRISTOL, GB, XP020090875 , Vol:16, Nr:12, S. 2852 - 2860 beschreibt ultrafeine Polyelektrolythydrogelfasern aus Polyacrylsäure/Polyvinylalkohol. Es wird die hitzeinduzierte Vernetzung der Fasern beschrieben. Das Dokument beschreibt Flächengebilde aus ultrafeinen Fasern mit Durchmessern zwischen 500 nm und 1,2 $\mu$m (dies entspricht etwa 0,0026 dtex bis 0,015 dtex). Kein Hinweis findet sich darauf, dass die Fasern einen Fasertiter von 0,5 bis 12 dtex aufweisen.

WO 2012/048768 beschreibt ein Verfahren zur Herstellung von PVA enthaltenden Fasern unter speziellen Temperbedingungen. Kein Hinweis findet sich auf Fasergebilde mit einem Flächengewicht von 20 bis 600 g/m$^2$.

Ahmet Cav et al. Journal of Applied Polymer Science, 20130915 Wiley, XP055073782, Vol:129, Nr:6, S. 3140 - 3149 beschreibt die Eigenschaften von elektroversponnenen Poly(vinylalkohol) Hydrogelnanofasern, die mit 1,2,3,4-Butantetracarbonsäure vernetzt sind. Das Dokument beschreibt Flächengebilde aus Nanofasern, kleiner 1000 nm (in etwa 0,01 dtex). Kein Hinweis findet sich darauf, dass die Fasern einen Fasertiter von 0,5 bis 12 dtex aufweisen. Die beschriebenen Flächengebilde weisen Aufnahmekapazitäten für Wasser sämtlich unter 4 g/g auf.

Darstellung der Erfindung

Die vorliegende Erfindung beschäftigt sich deshalb mit der Aufgabe, für Fasergebilde hergestellt aus wasserlöslichem Polyvinylalkohol, deren Verwendung, sowie ein zugehöriges Herstellungsverfahren und für Wundverbände oder Wundauflagen eine verbesserte Ausführungsform anzugeben, die sich insbesondere durch eine vereinfachte, kostengünstige Herstellung auszeichnen und eine unproblematische Weiterverarbeitung und/oder Anwendung ermöglichen. Zudem sollen aus den erfindungsgemäßen Fasergebilden hergestellte Wundverbände oder Wundauflagen eine erhöhte Stabilität, insbesondere eine hohe Höchstzugkraft und Höchstzugkraftdehnung im hydrogeliertem Zustand aufweisen, so dass sie in einem Stück wieder von der Wunde oder aus der Wundhöhle entfernt werden können.

Erfindungsgemäß wird diese Aufgabe durch die Gegenstände der unabhängigen Ansprüche gelöst. Vorteilhafte Ausführungsformen sind Gegenstand der abhängigen Ansprüche.

Überraschenderweise konnte festgestellt werden, dass Fasern oder Fasergebilde, die wasserlösliches Polyvinylalkohol enthalten, so durch Tempern behandelt werden können, dass sie mit wässrigen Lösungen oder Wundexsudat, insbesondere mit einer 0,9 prozentigen, wässrigen Natriumchlorid-Lösung (physiologische Kochsalzlösung) oder mit einer wässrigen Lösung gemäß der in DIN 13726-1 in Punkt 3.2.2.3 aufgeführten Prüflösung A ein stabiles Hydrogel ausbilden, welches eine sehr hohe Höchstzugkraft und Höchstzugkraftdehnung besitzt. Zudem weisen derartige Fasern oder Fasergebilde eine hohe Stabilität gegenüber Wasser oder wässrigen Lösungen auf. Ferner zeichnen sich die Fasern oder die erfindungsgemäßen Fasergebilde durch eine hohe Aufnahmekapazität und ein hohes Rückhaltevermögen für Wasser oder wässrige Lösungen, insbesondere 0,9 prozentige, wässrige Natriumchlorid-Lösung (physiologische Kochsalzlösung) oder eine wässrige Lösung gemäß der in DIN 13726-1 in Punkt 3.2.2.3 aufgeführten Prüflösung A aus.

In einem ersten Aspekt der Erfindung werden somit Fasergebilde, zwei-oder dreidimensional, vorgeschlagen, hergestellt aus Fasern aus einem ersten Faserrohmaterial, wobei das erste Faserrohmaterial wasserlöslichen Polyvinylalkohol und/oder Polyvinylalkohol-Copolymer enthält und wobei das Faserrohmaterial durch Tempern bei einer vorbestimmten Tempertemperatur, die größer ist als eine Glasübergangstemperatur und kleiner ist als eine Schmelz- oder Zersetzungstemperatur des verwendeten ersten Faserrohmaterials eine vorbestimmte Temperdauer lang vernetzt und hydrogelierend ausgebildet wird, wobei das Faserrohmaterial durch das Tempern vernetzt wird, wobei die Fasern aus dem ersten Faserrohmaterial einen Fasertiter von 0,5 bis 12 dtex aufweisen, und wobei die Fasergebilde eine Aufnahmekapazität für 0,9%ige, wässrige Natriumchlorid-Lösung oder Prüflösung A gemäß DIN 13726-1 Punkt 3.2.2.3 von 4-30 g/g aufweisen. Durch diese Behandlung wird das Faserrohmaterial stabilisiert und insbesondere auch die Fasern oder Fasergebilde, die aus dem Faserrohmaterial hergestellt sind, gegenüber wässrigen Lösungen stabilisiert, so dass sie in wässriger Lösung eine deutlich verringerte Löslichkeit zeigen. Dabei bilden die Fasern oder Fasergebilde gleichzeitig mit wässrigen Lösungen ein stabiles Hydrogel aus.

Unter Tempern im Sinne dieser Erfindung wird ein Prozess verstanden, bei dem das Faserrohmaterial, vorzugsweise in Form von Fasern oder Fasergebilden bei einer vorbestimmten Temperatur eine vorbestimmte Zeit, vorzugsweise bei Atmosphärendruck und Gasatmosphäre, insbesondere Luftatmosphäre, erhitzt werden. Zweckmäßigerweise wird das Faserrohmaterial in Form von Fasern oder eines Fasergebildes im trockenen Zustand, vorteilhafter Weise bei einer Restfeuchte von weniger als 10 Gew.%, noch bevorzugter von weniger als 5 Gew.%, noch bevorzugter von weniger als 3 Gew.% getempert. Zweckmäßigerweise werden die Fasern oder Fasergebilde zuerst auf die vorbestimmte Temperatur gebracht und dann die vorbestimmte Zeit lang auf dieser vorbestimmten Temperatur gehalten. Dabei können auftretende Temperaturschwankungen von zumindest +/-10%, insbesondere +/-5% und bevorzugt +/-1 % toleriert werden. Zudem kann beim Temperprozess beliebig viel Luft zugeführt oder abgeführt werden und im Temperbereich die Luft auf verschiedene Weise (z.B. Umluft, Durchluft) umgewälzt werden. Beim Temperprozess können auch andere Prozessgase wie Stickstoff oder Sauerstoff zusätzlich zugeleitet werden, um den Temperprozess und damit die Eigenschaften der Fasern oder Fasergebilde in erwünschter Weise zu beeinflussen.

Besonders bevorzugt wird der Temperprozess im Falle der zweidimensionalen Fasergebilde oder Vliesstoffe mit Durchluft in einem Bandtrockner durchgeführt. Mit Hilfe der Durchluft kann die Temperdauer im Vergleich zu der Temperdauer mit reiner Umluft auf ein Vielfaches reduziert werden.

Vorteilhaft können durch das Tempern die Fasern oder Fasergebilde so vernetzt werden, dass sie gegenüber Wasser eine höhere Löslichkeitsstabilität aufweisen. Darüber hinaus erhalten die Fasern oder Fasergebilde durch das Tempern die Fähigkeit, mit Wasser oder wässrigen Lösungen, insbesondere mit 0,9 prozentiger Natriumchlorid-Lösung oder mit einer Lösung gemäß der in DIN 13726-1 in Punkt 3.2.2.3 aufgeführten Prüflösung A, ein stabiles Hydrogel auszubilden, welches sich durch eine besonders hohe Höchstzugkraft und Höchstzugkraftdehnung auszeichnet.

Zudem können durch das Tempern Verunreinigungen oder Rückstände, wie zum Beispiel Spinnhilfsmittel, Avivage, Lösungsmittel oder dergleichen, signifikant reduziert oder sogar bis auf eine Konzentration unterhalb der jeweiligen Nachweisgrenze reduziert werden. Des Weiteren weisen die Fasern oder die erfindungsgemäßen Fasergebilde eine hohe Aufnahmekapazität und ein hohes Rückhaltevermögen (Retention) für Wasser, wässrige Lösungen, insbesondere für eine 0,9 Gew.%ige wässrige Natriumchlorid-Lösung oder für eine Lösung gemäß der in DIN 13726-1 in Punkt 3.2.2.3 aufgeführten Prüflösung A und/oder Wundexsudat auf.

So können die Fasern oder Fasergebilde für Wasser und/oder wässrige Lösungen eine Retention über 70 %, vorzugsweise von 70 % bis 100 % aufweisen. Im Fall von Fasern und/oder eindimensionalen, sowie zweidimensionalen Fasergebilden liegt bevorzugt eine relative Retention für 0,9 prozentige Natriumchlorid-Lösung oder für eine Lösung gemäß der in DIN 13726-1 in Punkt 3.2.2.3 aufgeführten Prüflösung A von über 70 % vor, noch bevorzugter über 80 %,- noch bevorzugter über 85 %, noch bevorzugter von 85 % bis 100%.

Erfindungsgemäß weist das Fasergebilde eine relative Aufnahmekapazität für 0,9 prozentige Natriumchlorid-Lösung oder für eine Lösung gemäß der in DIN 13726-1 in Punkt 3.2.2.3 aufgeführten Prüflösung A von 4 bis 30 g/g auf. Im Fall von zweidimensionalen Fasergebilden liegt bevorzugt eine relative Aufnahmekapazität für 0,9 prozentige Natriumchlorid-Lösung oder für eine Lösung gemäß der in DIN 13726-1 in Punkt 3.2.2.3 aufgeführten Prüflösung A von 4 bis 30 g/g vor, besonders bevorzugt von 4 bis 25 g/g,- noch bevorzugter von 5 bis 20 g/g, noch bevorzugter von 7 bis 20 g/g. Somit lassen sich vorteilhaft toxikologisch unbedenkliche und biokompatible Fasern oder Fasergebilde, sowie daraus herstellbaren Gele, insbesondere Hydrogele, erzeugen.

Folglich ist die eingangs genannte Aufgabe gelöst.

Man versteht unter Fasern ein im Verhältnis zu seiner Länge dünnes und flexibles Gebilde. Fasern weisen einen geringen Durchmesser auf und können miteinander durch dementsprechende Verfestigungsverfahren zu Fasergebilden aufgebaut werden. Somit kann ein Fasergebilde mehrere Fasern aufweisen. Man kann zwischen ein-, zweiund dreidimensionale Fasergebilden unterscheiden. Ein eindimensionales Fasergebilde hat im Vergleich zu seiner Länge eine geringe Breite und eine geringe Höhe. Ein zweidimensionales Fasergebilde hat im Vergleich zu seiner Länge und Breite eine geringe Höhe. Unter dreidimensionalen Fasergebilden sind Fasergebilde zu verstehen, die mehrere Lagen von zweidimensionalen Fasergebilden aufweisen. Dabei können die einzelnen Lagen des dreidimensionalen Fasergebildes durch nachfolgend beschriebene Verfestigungsverfahren oder anderweitig zueinander verbunden werden.

Aus Polymeren lassen sich mittels des Trocken- oder des Nassspinnverfahrens Filamente herstellen und mittels des Spinnvliesverfahrens Spinnvliese. Die Filamente können dabei als eindimensionale Fasergebilde angesehen werden, während die Spinnvliese zweidimensionale Fasergebilde darstellen können. Durch Schneiden und/oder Kräuseln der Filamente können Stapelfasern hergestellt werden, die als eindimensionale Fasergebilde eingeordnet werden können. Durch Garndrehung können aus Stapelfasern Stapelfasergarne hergestellt werden. Sie können als eindimensionale Fasergebilde verstanden werden. Aus Filamenten aufgebaute Garne können aus einem Filament (Monofilamentgarn) oder mehreren Filamenten (Multifilamentgarn) ausgebildet sein. Sie können ebenfalls als eindimensionale Fasergebilde angesehen werden. Mischgarne können durch Garnspinnen mehr als einer unterschiedlichen Stapelfaser bzw. Naturfaser hergestellt werden. Garne wie Naturfasergarne, Stapelfasergarne oder Filamentgarne oder Mischgarne, können mittels textiltechnischer Verfahren wie Weben, Stricken, Wirken, Sticken, Legen oder Nähen z.B. zu Geweben, Gestricken, Gelegen oder Gewirken weiterverarbeitet werden. Die Gewebe, Gestricke, Gelege bzw. Gewirke können als zweidimensionale Fasergebilde angesehen werden. Mittels vliestechnischer Verfahren wie Krempeln oder dem Airlaidverfahren können aus Stapelfasern Stapelfaservliese oder Airlaidvliese hergestellt werden, die ebenfalls als zweidimensionale Fasergebilde angesehen werden können. Erfindungsgemäß bevorzugt werden wasserlösliche Stapelfasern verwendet, die mittels Krempeln zu einem Stapelfaservlies gelegt werden.

Unverfestigte Vliese, z.B. Stapelfaser- oder Spinnvliese, können durch Verfestigungsverfahren zu Vliesstoffen verfestigt werden. Es kann beispielsweise als Verfestigungsverfahren ein Kalandrieren angewendet werden. Dabei werden die unverfestigten Vliese zwischen Rollen geführt, wobei auf den Rollen angeordnete Verschweißflächen in den Vliesen zumindest teilweise die Vliese durchdringende Verschweißungen erzeugen. Werden punktförmige Verschweißungen erzeugt, dann wird das Verfestigungsverfahren als PS (point seal)-Verfestigungsverfahren bezeichnet. Es ist aber auch die Ausbildung von linienförmigen Verschweißungen oder vollflächigen Verschweißungen möglich. Als weiteres Verfestigungsverfahren kann eine Heißluftverfestigung im Durchlufttrockner angewendet werden, wobei bei diesem Verfahren Verfestigungen durch Verschmelzungen an den Berührungspunkten der Fasern erzeugt werden. Des Weiteren ist ebenfalls der Einsatz von Bindern oder Bindemitteln denkbar, wobei hierbei die Fasern über Brücken aus Bindern oder Bindemittel miteinander verbunden werden. Es können insbesondere auch mechanische Verfestigungsverfahren zum Einsatz kommen, wie z.B. das Nadelverfestigungsverfahren, bei dem die Verfestigung mittels Nadeln vorgenommen wird. Des Weiteren ist ebenfalls Walken oder Filzen oder dergleichen denkbar. Dabei kann auch eine Kombination von mehreren Verfestigungsverfahren angewendet werden. Bevorzugt werden das Nadelverfestigungsverfahren und/oder das PS-Verfestigungsverfahren angewendet.

Durch das Tempern können die wasserlöslichen Fasern aus Polyvinylalkohol oder die Fasergebilde, die die wasserlöslichen Fasern aus Polyvinylalkohol enthalten, vernetzt werden. Somit können sowohl die Fasern selbst als auch die Fasergebilde durch Tempern derartig verändert werden, dass sie eine höhere Stabilität gegenüber Wasser, insbesondere gegenüber einer 0,9 prozentigen, wässrigen Natriumchlorid-Lösung oder gegenüber einer Lösung gemäß der in DIN 13726-1 in Punkt 3.2.2.3 aufgeführten Prüflösung A aufweisen

Die getemperten Fasern oder die daraus hergestellten Fasergebilde weisen vorzugsweise einen löslichen Anteil von 1% bis 30%, vorzugsweise von 1% bis 25%, noch bevorzugter von 1% bis 20% und noch bevorzugter von 1% bis 15% in 0,9% iger, wässriger Natriumchlorid-Lösung oder in einer Lösung gemäß der in DIN 13726-1 in Punkt 3.2.2.3 aufgeführten Prüflösung A auf.

Darüber hinaus wird den Fasern oder dem Fasergebilde durch das Tempern in vorteilhafter Weise die Eigenschaft verliehen mit Wasser bzw. den oben genannten Lösungen ein stabiles Hydrogel mit hoher Höchstzugkraft und Höchstzugkraftdehnung auszubilden. Unter "hydrogelierend" ist die Fähigkeit zur Ausbildung eines Hydrogels zu verstehen, das als flüssige Phase Wasser oder eine wässrige Lösung, besonders bevorzugt eine 0,9 prozentige, wässrige Natriumchlorid-Lösung oder eine Lösung gemäß der in DIN 13726-1 in Punkt 3.2.2.3 aufgeführten Prüflösung A, aufweist.

Ein Hydrogel ist ein in Wasser gequollenes, hydrophiles polymeres Netzwerk. Insbesondere ist unter einem Hydrogel ein System aus mindestens einer festen und einer flüssigen Phase zu verstehen, wobei die feste Phase ein dreidimensionales Netzwerk ausbildet, dessen Poren durch wässrige Lösung ausgefüllt werden können und dadurch quellen. Beide Phasen können sich dabei vollständig durchdringen und demzufolge kann ein Gel im Vergleich zu einem Schwamm eine flüssige Phase stabiler gegenüber z.B. Druck speichern. Darüber hinaus besitzt ein Hydrogel ein hohes Rückhaltevermögen (Retention) für wässrige Lösungen.

Die Fasern oder die erfindungsgemäßen Fasergebilde sind hydrogelierend ausgebildet und weisen demzufolge ein hervorragendes Bindungs- und Rückhaltevermögen für wässrige Phasen auf. Sie werden bevorzugt trocken auf die Wunde aufgelegt oder Wundhöhlen damit ausgefüllt. Sie bilden mit dem Wundexsudat stabile Hydrogele aus und schaffen somit ein optimales Wundklima zur Wundheilung ohne mit der Wunde zu verkleben. Eine derartige feuchte Wundbehandlung kann den Heilungsprozess unterstützen. Durch die hohe Höchstzugkraft und Höchstzugkraftdehnung des mit dem Wundexsudat ausgebildeten Hydrogels, lassen sich die Fasern oder Fasergebilde in einem Stück aus der Wunde oder Wundhöhle entfernen.

Ebenfalls für die feuchte Wundbehandlung können die Fasern oder die erfindungsgemäßen Fasergebilde mit einer flüssigen Phase bestückt in hydrogelierter Form eingesetzt werden. Bevorzugt wird dabei als flüssige Phase Wasser verwendet und besonders bevorzugt eine 0,9 prozentige, wässrige Natriumchlorid-Lösung, Ringer-Lösung oder wirkstoffhaltige Lösungen oder eine Lösung gemäß der in DIN 13726-1 in Punkt 3.2.2.3 aufgeführten Prüflösung A.

Polyvinylalkohole sind Polymere und können durch Hydrolyse aus Polyvinylacetat hergestellt werden. Die technischen Eigenschaften des Polyvinylalkohols, wie insbesondere seine Wasserlöslichkeit hängen unter anderem vom Herstellungsverfahren, von der Molmasse und dem verbleibenden Anteil an Acetyl-Gruppen ab (Hydrolysegrad). Mit abnehmender Molmasse und Hydrolysegrad nimmt die Löslichkeit in Wasser zu. Je nach Molmasse und Hydrolysegrad besitzen die Polyvinylalkohole eine unterschiedliche Wasserlöslichkeit. So lösen sich einige Typen von Polyvinylalkohol erst bei einer erhöhten Temperatur in Wasser auf (z.B. ab 90°C). Fasern aus Polyvinylalkohol werden bei ihrer Herstellung üblicherweise auf ein Mehrfaches ihrer ursprünglichen Länge verstreckt und können hierbei auch erhitzt werden (Verstreckungstemperatur), um die Kristallinität und Festigkeit der Fasern zu erhöhen. Dabei werden durch Parallelorientierung der Molekülketten die Ausbildung von intermolekularen Wasserstoffbrückenbindungen ermöglicht. Damit kann auch die Wasserlöslichkeit der Polyvinylalkohol-Fasern eingestellt werden.

Die als erstes Faserrohmaterial verwendeten ungetemperten Fasern aus Polyvinylalkohol können erfindungsgemäß in einem Überschuss von Wasser bereits unterhalb einer Temperatur von 50°C, bevorzugt unterhalb 40°C, besonders bevorzugt unterhalb 30°C, noch bevorzugter unterhalb von 25°C wasserlöslich sein, wobei die ungetemperten Fasern selbstverständlich auch oberhalb dieser Werte wasserlöslich sein können. Ferner können die ungetemperten Fasern auch oberhalb von 15°C und/oder oberhalb von 20°C wasserlöslich sein. Insbesondere können die ungetemperten Fasern in einem Bereich zwischen 0°C und 150 °C oder zwischen 5°C und 100 °C oder zwischen 10°C und 100 °C, oder zwischen 15°C und 100 °C oder zwischen 20°C und 100 °C wasserlöslich sein, wobei unter wasserlöslich zu verstehen ist, dass sich die Fasern in einem Überschuss von Wasser zumindest zu 70 %, vorzugsweise zu mehr als 80 %, noch bevorzugter zu mehr als 90 % und insbesondere zu mehr als 95 %, und insbesondere zu 100 % auflösen.

Das Polyvinylalkohol, das für die Herstellung der Fasern aus Polyvinylalkohol verwendet wird, kann dabei durch Copolymerisation mit anderen Monomeren (z.B. Polyethylen-Vinylalkohol) oder durch den Einbau von funktionellen Gruppen modifiziert sein, wodurch weitere physikalische wie auch chemische Eigenschaften gegebenenfalls gezielt in die Fasern eingebaut werden. So ist im Fall der Verwendung von beispielsweise Polyethylen-Vinylalkohol die Anzahl an OH-Gruppen reduziert ausgebildet.

Bevorzugt können als Polyvinylalkohol-Copolymere Polyethylen-Vinylalkohol, Polyvinylalkohol-Styrol, Polyvinylalkohol-Vinylacetat, Polyvinylalkohol-Vinylpyrrolidon, Polyvinylalkohol-ethylenglykol und/oder Polyvinylalkohol, besonders bevorzugt Polyethylen-Vinylalkohol, Polyvinylalkohol-Vinylacetat, Polyvinylalkohol-Vinylpyrrolidon, Polyvinylalkohol-Vinylamin, Polyvinylalkohol-Acrylat, Polyvinylalkohol-Acrylamid, Polyvinylalkohol-ethylenglykol eingesetzt werden. Die Polyvinylalkohol-Copolymere können als Blockcopolymere und/oder Pfropfcopolymere und/oder Block- Pfropfcopolymere, statistische oder alternierende Systeme und jegliche Mischungen untereinander vorliegen. Der Anteil an anderen Monomereinheiten im Polyvinylalkohol beträgt dabei maximal 30Gew.%, vorzugsweise 1 bis 30 %, noch bevorzugter 5 bis 15 %, jeweils bezogen auf jeweils bezogen auf die Gesamtanzahl an Monomereinheiten im Polyvinylalkohol-Copolymer.

Es können aber auch andere funktionelle Gruppen in das Polvinylalkohol und/oder in die Fasern oder in das Fasergebilde z.B. durch Substitution oder polymeranaloge Reaktionen eingebracht werden. Als funktionelle Gruppen kommen hierbei insbesondere Carbonsäuren, ungesättigte Carbonsäuren wie Methyacrylsäuren, Acrylsäuren, Peroxycarbonsäuren, Sulfonsäuren, Carbonsäureester, Sulfonsäureester, Aldehyde, Thioaldehyde, Ketone, Thioketone, Amine, Ether, Thioether, Isocyanate, Thiocyanate, Nitrogruppen in Betracht. Der Anteil an anderen funktionellen Gruppen im Polyvinylalkohol beträgt dabei maximal 30 %, vorzugsweise 1 bis 30 %, noch bevorzugter 5 bis 15 %, jeweils bezogen auf die Anzahl an OH-Gruppen im Polyvinylalkohol .

Des Weiteren kann das erste Faserrohmaterial als physikalische Mischung zwischen dem wasserlöslichen Polyvinylalkohol und mindestens einem anderem Polymer (Polymer-Blend) ausgebildet sein. Der Anteil an wasserlöslichem Polyvinylalkohol beträgt dabei im Polymer-Blend mindestens 70 Gew.%, bezogen auf die Gesamtmasse des Polymer-Blends.

Vorteilhaft weist der daraus resultierende Polymer-Blend im Vergleich zu den eingesetzten Polymeren unterschiedliche physikalische Eigenschaften und gegebenenfalls auch chemische Eigenschaften auf. Üblicherweise sind dabei die Eigenschaften des Polymer-Blends eine Summe der Eigenschaften der verwendeten Polymere. Somit kann durch den Einsatz von Polymer-Blends eine Auswahl an ersten Faserrohmaterialien weiter vergrößert werden.

Dabei können zur Ausbildung eines derartigen Polymer-Blends gelierende weitere Polymere, wie z.B. Alginate, Celluloseether, wie Carboxymethylcellulosen, Methyl-, Ethylcellulosen, Hydroxymethylcellulosen, Hydroxyethylcellulosen, Hydroxyalkylmethylcellulosen, Hydroxypropylcellulosen, Celluloseester, wie Celluloseacetat, oxidierte Cellulosen, bakterielle Cellulosen, Cellulosecarbonate, Gelatinen, Kollagene, Stärken, Hyaluronsäuren, Pektine, Agar, Polyacrylate, Polyvinylamine, Polyvinylacetate, Polyethylenglycole, Polyethylenoxide, Polyvinylpyrrolidone, Polyurethane oder nichtgelierende weitere Polymere, wie z.B. Polyolefine, Cellulose, Cellulosederivate, regenerierte Cellulose wie Viskose, Polyamide, Polyacrylnitrile, Polyvinylchloride, Chitosane, Polylactide, Polyglykolide, Polyesteramide, Polycaprolactone, Polyhexamethylenterephthalate, Polyhydroxybutyrate, Polyhydroxyvalerate oder Polyester eingesetzt und zu dem wasserlöslichen Polyvinylalkohol hinzu gemischt werden. Die oben aufgeführten Blends können als Homopolymere oder Copolymere eingesetzt werden. Es können auch Blockcopolymere und/oder Pfropfcopolymere und/oder Block-Pfropfcopolymere, statistische oder alternierende Systeme und jegliche Mischungen untereinander eingesetzt werden.

Unter Alginate versteht man die Salze der Alginsäure, einem natürlichen in Algen vorkommenden Polymer, der beiden Uronsäuren $\alpha$-L-Guluronsäure und $\beta$-D-Mannuronsäure, die 1,4-glycosidisch verknüpft sind. Dabei wird von dem Begriff Alginat E401, E402, E403, E404 und E405 (PGA) umfasst. Von dem Begriff Polyolefine wird PE, PB, PIB und PP umfasst. Von dem Begriff Polyamiden werden PA6, PA6.6, PA6/6.6, PA6.10, PA6.12 PA69, PA612, PA11, PA12, PA46, PA1212 und PA6/12 umfasst. Von dem Begriff Cellulose wird auch regenerierte Cellulose wie Viskose, sowie Cellulosederivate und chemisch und/oder physikalisch modifizierte Cellulose umfasst. Von dem Begriff Polyester werden PBT, BC, PET, PEN und UP umfasst.

Das Polyvinylalkohol, das für die Herstellung der Fasern aus Polyvinylalkohol verwendet wird oder aus dem die Polyvinylalkohol-Fasern bestehen, kann mit verschiedenen Hydrolysegraden und mittleren Molmassen zum Einsatz kommen.

Der Hydrolysegrad des Polyvinylalkohols beträgt insbesondere mehr als 70%, bevorzugt oberhalb von 75%, noch bevorzugter oberhalb von 80% und bis zu 100 %.

Das Massenmittel der Molmasse des Polyvinylalkohols liegt insbesondere im Bereich von 20000 bis 200000 g/mol, bevorzugt im Bereich von 30000 bis 170000 g/mol, besonders bevorzugt im Bereich von 40000 bis 150000 g/mol, noch bevorzugter im Bereich von 50000 bis 140000 g/mol, noch bevorzugter im Bereich von 70000 bis 120000 g/mol.

Das Zahlennmittel der Molmasse des Polyvinylalkohols liegt insbesondere im Bereich von 10000 bis 120000 g/mol, bevorzugt im Bereich von 20000 bis 100000 g/mol, besonders bevorzugt im Bereich von 20000 bis 80000 g/mol, noch bevorzugter im Bereich von 25000 bis 70000 g/mol.

Erfindungsgemäß werden Fasern aus einem ersten Faserrohmaterial mit einem Fasertiter von 0,5 bis 12 dtex eingesetzt. Bevorzugt werden sie mit einem Fasertiter von 1 bis 8 dtex eingesetzt, besonders bevorzugt mit einem Fasertiter von 1,4 bis 7 dtex und noch bevorzugter mit einem Fasertiter von 1,4 bis 4 dtex. Dabei ist unter dtex oder Dezitex das Gewicht in Gramm der Faser bei einer ggf. theoretischen Länge von 10.000 m zu verstehen. Fasern mit einem Einzeltiter von kleiner als 0,5 dtex sind weniger geeignet.

Die Fasern aus einem ersten Faserrohmaterial können eine Länge von 30 bis 100 mm aufweisen. Bevorzugt werden sie mit einer Länge von 30 bis 90 mm eingesetzt, besonders bevorzugt mit einer Länge von 30 bis 80 mm und noch bevorzugter mit einer Länge von 35 bis 70 mm.

Insbesondere handelt es sich bei den Fasern aus dem ersten Faserrohmaterial um sogenannte Stapelfasern, die für die Herstellung von Stapelfaservliesstoffen verwendet werden.

Außerdem können die Fasern oder Fasergebilde zusätzlich weitere Fasern aus zumindest einem zweiten Faserrohmaterial aufweisen. Dabei kann das zweite Faserrohmaterial nicht-gelierend oder gelierend ausgebildet sein. Somit können als weitere Fasern nicht-gelierende oder gelierende Fasern eingesetzt werden.

Vorteilhaft kann durch den Einsatz von weiteren Fasern ein gewünschtes Verhalten der Fasern oder der Fasergebilde gezielt verbessert werden. So kann durch den Einsatz der weiteren Fasern die Aufnahmekapazität der Fasergebilde noch weiter erhöht und der Schrumpf des Fasergebildes in wässriger Lösung reduziert werden.

Als weiteres Faserrohmaterial für die weiteren Fasern kann Polyester, wie Polyethylenterephthalat, wasserunlösliches Polyvinylalkohol, wasserlösliches Polyvinylalkohol, das oberhalb einer Temperatur von 50°C wasserlöslich ist, Polyolefine, wie Polyethylen oder Polypropylen, Cellulose, Cellulosederivate, regenerierte Cellulose, wie Viskose, Polyamide, Polyacrylnitrile, Chitosane, Elastane, Polyvinylchloride, Polylactide, Polyglykolide, Polyesteramide, Polycaprolactone, pflanzliche Naturfasern, Alginate, modifiziertes Chitosan, Celluloseether, wie Carboxymethylcellulosen, Methyl-, Ethylcellulosen, Hydroxymethylcellulosen, Hydroxyethylcellulosen, Hydroxyalkylmethylcellulosen, Hydroxypropylcellulosen, Celluloseester, wie Celluloseacetat, oxidierte Cellulosen, bakterielle Cellulosen, Cellulosecarbonate, Gelatinen, Kollagene, Stärken, Hyaluronsäuren, Pektine, Agar, Polyvinylamine, Polyvinylacetate, Polyethylenglycole, Polyethylenoxide, Polyvinylpyrrolidone, Polyurethane und/oder Polyacrylate eingesetzt werden. Die aufgeführten zweiten Faserrohmaterialien können sowohl als Homopolymere als auch als Copolymere eingesetzt werden. Es können auch Blockcopolymere und/oder Pfropfcopolymere und /oder Block-Pfropfcopolymere, statistische oder alternierende Systeme und jegliche Mischungen untereinander eingesetzt werden.

Es ist auch der gleichzeitige Einsatz von gelierenden und nicht-gelierenden weiteren Fasern oder von Mischungen aus verschiedenen weiteren Fasern möglich. Bevorzugt ist dabei die Verwendung von weiteren Fasern aus Polyamid, Polyester, wasserunlöslichem Polyvinylalkohol oder Polyvinylalkohol, welches sich oberhalb einer Temperatur von 50°C löst, Polyacrylat, Polyacrylsäure und noch bevorzugter aus Polyester oder wasserunlösliches Polyvinylalkohol oder Polyvinylalkohol, welches sich oberhalb einer Temperatur von 50°C löst und/oder Mischungen hiervon.

Die weiteren Fasern können auch aus einem als Polymer-Blend ausgebildeten zweiten Faserrohmaterial hergestellt sein. Dabei ergeben sich für die weiteren Fasern die schon vorhergehend bei dem ersten Faserrohmaterial aufgezeigten Vorteile.

Die Fasern aus dem erstem Faserrohmaterial oder aus dem weiteren Faserrohmaterial können auch in Form einer Bikomponentenfaser und/oder Mehrkomponentenfaser eingesetzt werden. Dabei können die Bikomponentenfasern und/oder Mehrkomponentenfasern in geometrischen Formen wie "Core shell", "Side-by-Side", "Pie- oder Orangetype", "Matrix with fibrils" vorliegen.

Die Bikomponentenfasern und/oder Mehrkomponentenfasern des weiteren Faserrohmaterials können zur thermischen Verfestigung der Vliese genutzt werden. Bei Erwärmung dieser Fasern erfolgt eine thermische Bindung des Vlieses. Beispielsweise schmilzt bei einer Core shell-Faser der shell-Anteil und verfestigt somit das Vlies. Als Bikomponentenfasern und/oder Mehrkomponentenfasern können Fasern aus dem weiteren Faserrohmaterial aus Polyethylen/Polypropylen, Polyethylen/Polyester, CoPolyester/Polyethylenterephthalat, Polyamid6/ Polyamid6.6, Polybutylenterephthalat/Polyethylenterephthalat eingesetzt werden.

Vorteilhaft kann durch die Verwendung von weiteren Fasern die Aufnahmekapazität von Wasser, insbesondere von einer 0,9 prozentigen Natriumchlorid-Lösung oder von einer Lösung gemäß der in DIN 13726-1 in Punkt 3.2.2.3 aufgeführten Prüflösung A, im Vergleich zu Fasergebilden ohne weitere Fasern deutlich erhöht werden, da insbesondere mittels der nicht-gelierenden Fasern ein Gel-Blocking-Effekt, der ab einer vorbestimmten Sättigung eine weitere Aufnahme von Wasser, insbesondere von einer 0,9 prozentigen Natriumchlorid-Lösung oder von einer Lösung gemäß der in DIN 13726-1 in Punkt 3.2.2.3 aufgeführten Prüflösung A, verhindert, verringert werden kann. Zudem kann der Schrumpf der Fasergebilde, die Fasern aus dem erstem Faserrohmaterial enthalten, in wässriger Lösung durch Beimengen von weiteren Fasern deutlich verringert werden.

Dabei kann der Schrumpf von zumindest zweidimensionalen Fasergebilden durch Ausstanzen von 10,0 cm x 10,0 cm (Fläche1) große Stücken und Eintauchen derselben in eine 0,9 %ige wässrige Natriumchlorid-Lösung oder eine Lösung gemäß der in DIN 13726-1 in Punkt 3.2.2.3 aufgeführten Prüflösung A bestimmt werden. Die ausgestanzten und getränkten Stücke werden aus der Lösung entnommen und für 2min abgetropft. Danach wird die Größe der Stücke abgemessen (Fläche 2). Der Schrumpf der Vliesstoffe kann dann nach folgender Formel berechnet werden:

$$Schrumpf\ [\%] = 100 - \frac{Fläche2\ [cm^2]}{Fläche1\ [cm^2]} * 100$$

Der Anteil an weiteren Fasern in den Fasergebilden kann 1 bis 70 Gew.% betragen. Bevorzugt beträgt der Anteil 1 bis 65 Gew.%, besonders bevorzugt 5 bis 60 Gew.%, noch bevorzugter 10 bis 50 Gew.%, noch bevorzugter zwischen 15 und 40 Gew.%.

Die weiteren Fasern können einen Fasertiter von 0,5 bis 12 dtex aufweisen. Bevorzugt werden sie mit einen Fasertiter von 1 bis 8 dtex eingesetzt, besonders bevorzugt mit einen Fasertiter von 1,4 bis 7 dtex und noch bevorzugter mit einen Fasertiter von 1,4 bis 4 dtex. Dabei ist unter dtex oder Dezitex das Gewicht in Gramm der Faser bei einer ggf. theoretischen Länge von 10.000 m zu verstehen. Fasern mit einem Einzeltiter von kleiner als 0,5 dtex sind weniger geeignet.

Die weiteren Fasern können eine Länge von 30 bis 100 mm aufweisen. Bevorzugt werden sie mit einer Länge von 30 bis 90 mm eingesetzt, besonders bevorzugt mit einer Länge von 30 bis 80 mm und noch bevorzugter mit einer Länge von 35 bis 70 mm.

Insbesondere handelt es sich bei den weiteren Fasern aus dem weiterem Faserrohmaterial um sogenannte Stapelfasern, die für die Herstellung von Stapelfaservliesstoffen verwendet werden.

Des Weiteren können die Fasern oder die Fasergebilde zusätzlich Additive aufweisen. Dabei können als Additive pharmakologische Wirkstoffe oder Medikamente, wie Antibiotika, Analgetika, Antiinfektiva, entzündungshemmende Mittel, wundheilungsfördernde Mittel oder dergleichen, antimikrobielle, antibakterielle oder antivirale Agentien, blutstillende Mittel, Enzyme, Aminosäuren, Antioxidantien, Peptide und/oder Peptidsequenzen, Polysaccharide (z.B. Chitosan), Wachstumsfaktoren (z.B. Purine, Pyrimidine), lebende Zellen, Tricalciumphosphat , Hydroxyapatit, insbesondere speziell Hydroxyapatitnanopartikel, geruchsadsorbierende Additive wie Aktivkohle, Cyclodextrine, Metalle wie Silber, Gold, Kupfer, Zink, Kohlenstoffverbindungen, wie Aktivkohle, Graphit oder dergleichen, kosmetische Wirkstoffe, Vitamine und/oder Verarbeitungshilfsstoffe wie oberflächenaktive Substanzen, Netzmittel, Avivagen, Antistatika eingesetzt werden

Durch den Einsatz von zumindest einem Additiv können die Fasern oder Fasergebilde zudem vorteilhaft mit weiteren physikalischen, chemischen sowie biologischen Eigenschaften ausgestattet werden. So ermöglicht beispielsweise eine Ausrüstung der Fasern oder Fasergebilde mit Silber oder Silbersalzen oder antimikrobiellen Agentien wie Polyhexanid (Polyhexamethylenbiguanid), Chlorhexidin, Cetylpyridiniumchlorid, Benzalkoniumchlorid, Medihoney, PVPlod, Wasserstoffperoxid, 8-Chinolinol, Chloramin, Ethacridinlactat, Nitrofural,oder Octenidin (N-Octyl-1-[10-(4-octyliminopyridin-1-yl)decyl]pyridin-4-imin) eine antibakterielle Wirkung der Fasern oder Fasergebilde.

Beispielsweise können die Fasern oder Fasergebilde mit einer ethanolischen Lösung, die ein antimikrobielles Agens enthält, ausgerüstet werden. Bevorzugt werden die Fasern oder Fasergebilde mit einer ethanolischen Lösung, die ein antimikrobielles Agens wie Polyhexanid, Octenidin oder Silbersalze enthält, mittels eines Foulards ausgerüstet. Es kommen jedoch auch jegliche andere Beschichtungsverfahren in Betracht. Zudem können die Fasern oder Fasergebilde mit einer wässrigen Lösung, die das antimikrobielle Agens enthält, ausgerüstet werden. Bevorzugt wird bei dem Auftrag aus wässriger Lösung eine kontrollierte Menge an Wasser eingesetzt, unter der die Fasern oder Fasergebilde nicht irreversibel hydrogelieren und sich in ihrer morphologischen Struktur verändern. Insbesondere kommen hier Beschichtungsverfahren wie der Schaumauftrag, Kisscoater oder dergleichen in Betracht.

Die erfindungsgemäßen Fasergebilde weisen ein Flächengewicht von 20 bis 600 g/m$^2$auf. Im Fall von zweidimensionalen Fasergebilden liegt ein Flächengewicht von 20 bis 600 g/m$^2$, bevorzugt von 50 bis 500 g/m$^2$, noch bevorzugter von 70 bis 450 g/m$^2$, noch bevorzugter von 80 bis 350 g/m$^2$, noch bevorzugter von 80 bis 250 g/m$^2$, noch

bevorzugter von 90 bis 220 g/m$^2$, noch bevorzugter von 100 bis 200 g/m$^2$ vor.

Im Fall von zweidimensionalen Fasergebilden liegt die Dicke des Fasergebildes vorzugsweise im Bereich von 0,2 bis 10 mm, bevorzugt im Bereich von 0,5 bis 8 mm, noch bevorzugter im Bereich von 0,7 bis 7 mm, noch bevorzugter im Bereich von 0,8 mm bis 6 mm, noch bevorzugter im Bereich von 0,9 bis 5 mm, besonders bevorzugt im Bereich von 1,0 bis 4 mm.

Im Fall von zweidimensionalen Fasergebilden werden diese bevorzugt thermisch oder mechanisch verfestigt. Besonders bevorzugt werden diese mechanisch durch Vernadelung verfestigt. Dabei liegt die Einstichdichte bevorzugt im Bereich von 70 bis 200 Einstichen pro Quadratzentimeter, besonders bevorzugt im Bereich von 70 bis 170 Einstichen pro Quadratzentimeter, besonders bevorzugt im Bereich von 80 bis 150 Einstichen pro Quadratzentimeter, besonders bevorzugt im Bereich von 100 bis 150 Einstichen pro Quadratzentimeter.

Die erfindungsgemäßen Fasergebilde können eine besonders hohe Höchstzugkraft sowohl in Längs- als auch in Querrichtung des Fasergebildes im hydrogeliertem Zustand aufweisen. Beispielsweise weisen erfindungsgemäße Fasergebilde, die ein Flächengewicht von 140 bis 220 g/m$^2$ haben und mechanisch durch Vernadelung beispielsweise mit einer-Einstichdichte von 100-150 Einstichen pro Quadratzentimeter verfestigt wurden, eine Höchstzugkraft im hydrogelierten Zustand von oberhalb 0,3 N/2cm auf. Die bevorzugte Höchstzugkraft im hydrogeliertem Zustand liegt oberhalb von 0,4 N/2cm, noch bevorzugter oberhalb von 0,5 N/2cm noch bevorzugter oberhalb von 0,8 N/2cm, noch bevorzugter oberhalb von 1,0 N/2cm, noch bevorzugter oberhalb von 1,5 N/2cm, noch bevorzugter oberhalb von 2,0 N/2cm und/oder unterhalb von 50 N/2cm, und/oder unterhalb von 40 N/2cm, und/oder unterhalb von 35 N/2cm. Dementsprechend liegt eine Höchstzugkraft im hydrogelierten Zustand bevorzugt im Bereich von 0,3 N/2cm bis 50 N/2cm, noch bevorzugter von 0,4 N/2cm bis 40 N/2cm, noch bevorzugter von 0,5 N/2cm bis 30 N/2cm, noch bevorzugter von 0,8 N/2cm bis 25 N/2cm, noch bevorzugter von 1 N/2cm bis 25 N/2cm, noch bevorzugter von 1,5 N/2cm bis 25 N/2cm, noch bevorzugter von 2 N/2cm bis 25 N/2cm.

Die erfindungsgemäßen Fasergebilde können eine besonders hohe Höchstzugkraftdehnung sowohl in Längs- als auch in Querrichtung des Fasergebildes im hydrogeliertem Zustand aufweisen. Die bevorzugte Höchstzugkraftdehnung im hydrogeliertem Zustand liegt bei 20 bis 300%, besonders bevorzugt bei 30 bis 250% noch bevorzugter bei 50 bis 200%, noch bevorzugter bei 70 bis 200%, noch bevorzugter bei 80 bis 200%, noch bevorzugter bei 90 bis190%, noch bevorzugter bei 90 bis 180%. Beispielsweise weisen erfindungsgemäße Fasergebilde, die ein Flächengewicht von 140 bis 220 g/m$^2$ haben und mechanisch durch Vernadelung beispielsweise mit einer Einstichdichte von 100-150 Einstichen pro Quadratzentimeter verfestigt wurden, die oben genannten Höchstzugkraftdehnungswerte auf.

In einem weiteren Aspekt der Erfindung wird ein Verfahren zur Herstellung von hydrogelierend ausgebildeten Fasergebilden vorgeschlagen, in dem Fasern oder Fasergebilde aus einem ersten wasserlöslichen Faserrohmaterial umfassend Polyvinylalkohol und/oder unsubstituiertes oder teilweise unsubstituiertes Polyvinylalkohol-Copolymer bei einer vorbestimmten Tempertemperatur, die größer als eine Glasübergangstemperatur und/oder kleiner ist als eine Schmelztemperatur des verwendeten ersten Faserrohmaterials, eine vorbestimmte Temperdauer lang getempert werden, so dass die Fasern vernetzt werden.

Vorteilhaft können durch diesen sehr einfachen Prozess Fasergebilde hergestellt werden, die hydrogelierende Eigenschaften aufweisen. Dabei ist nur ein einziger Prozessschritt zur Stabilisierung der Fasern oder Fasergebilde notwendig, der zudem derart umweltfreundlich ausgebildet ist, dass keinerlei Lösungsmittel, Neben- oder Abfallprodukte anfallen. Zudem können durch das Tempern gegebenenfalls in den Fasern oder Fasergebilde enthaltene Verunreinigungen, wie zum Beispiel Avivage, Spinnhilfsmittel oder Lösungsmittel, entfernt werden.

Die vorbestimmte Tempertemperatur wird so gewählt, dass sie größer ist als die Glasübergangstemperatur des verwendeten ersten Faserrohmaterials. Zudem wird die vorbestimmte Tempertemperatur derart gewählt, dass sie kleiner ist, als eine Schmelztemperatur des verwendeten ersten Faserrohmaterials. Werden dabei mehrere Fasern aus unterschiedlichem Faserrohmaterial verwendet, so wird die vorbestimmte Temperatur vorzugsweise so gewählt, dass sie unterhalb der Schmelztemperatur oder Zersetzungstemperatur bevorzugt aller verwendeten Faserrohmaterialien liegt.

In vielen Anwendungsfällen haben sich Tempertemperaturen in einem Temperaturbereich von 85 bis 220 °C, besonders bevorzugt von 100 bis 200 °C, noch bevorzugter von 120°C bis 190 °C, noch bevorzugter zwischen 130°C und 180°C, ganz besonders bevorzug zwischen 140°C und 180°C, noch bevorzugter zwischen 150°C und 175°C als zweckmäßig erwiesen.

Die vorbestimmte Temperdauer kann von 10 min bis 14h betragen. Bevorzugt beträgt die Temperdauer von 10 min bis 10h, besonders bevorzugt von 10 min bis 8h, noch bevorzugter von 10 min bis 7h, noch bevorzugter von 10 min bis 6h, noch bevorzugter von 10 min bis 5h, noch bevorzugter zwischen 20 min und 5h, noch bevorzugter 30 min bis 5h, noch bevorzugter 30 min bis 4h.

Durch die Auswahl derartiger Tempertemperaturen und Temperzeiten kann das erfindungsgemäße Vernetzen der Fasern oder Fasergebilde besonders schonend für die Fasern oder Fasergebilde durchgeführt werden. Zudem können durch die Auswahl dieser Temperbedingungen die Eigenschaften der Fasern oder Fasergebilde optimal eingestellt werden. So besitzen die Fasern oder Fasergebilde durch Auswahl dieser Temperbedingungen eine hohe Absorptionskapazität und Rückhaltevermögen (Retention) sowie eine sehr hohe Höchstzugkraft und Höchstzugkraftdehnung im hydrogeliertem Zustand. Durch Variation der Tempertemperaturen und Temperzeiten ist eine unterschiedlich ausgebildete Vernetzung steuerbar, so dass die vernetzten Fasern oder Fasergebilde gegebenenfalls unterschiedliche Eigenschaften aufweisen. Auch können durch die Auswahl dieser Temperbedingungen aus den Fasern oder Fasergebilden ggf. vorhandene Verunreinigungen wie Lösungsmittelrückstände oder Faserhilfsmittel und Faserverarbeitungshilfsmittel wie Avivagen, Netzmittel, Antistatika sogar bis auf einen nicht mehr nachweisbaren Gehalt reduziert werden. Dies ist insbesondere für die Verwendung der Fasern oder Fasergebilde in Wundauflagen von Vorteil, da die oben genannten Verunreinigungen oder Faserhilfsmittel /-verarbeitungsmittel toxikologisch bedenklich sein können.

Insbesondere vor oder nach dem Tempern kann ein Verfahren angewendet werden, um zwei- oder dreidimensionale Fasergebilde zu erhalten. Dabei kann aus den Fasern z.B. mittels eines vorhergehend beschriebenen Verfahrens das jeweilige Fasergebilde hergestellt werden.

Vorteilhaft können durch ein derartiges Verfestigungsverfahren die Fasern oder Fasergebilde in eine gewünschte Form gebracht werden und in dieser Form verfestigt werden.

Zudem können auch weitere Fasern aus zumindest einem zweiten Faserrohmaterial zugemischt werden.

Des Weiteren kann eine Nachbehandlung durchgeführt werden. Zudem ist eine Zumischung von Verarbeitungshilfsstoffen, insbesondere vor dem Verfestigungsverfahren möglich. Ebenfalls eine Zugabe von z.B. zuvor beschriebenen Additiven kann vorgenommen werden.

Als mögliche Nachbehandlung kann eine Nachverfestigung, eine Sterilisierung wie z.B. Strahlensterilisation oder Sterilisation mit Ethylenoxid, eine Bestrahlung, eine Beschichtung, eine Ausrüstung, eine Applikation von Avivagen, eine chemische Modifizierung oder eine Weiterverarbeitung wie z.B. Rascheln, Einbringen von Verstärkungsfasern vorgenommen werden.

Eine besonders bevorzugte Nachbehandlung der Fasern oder Fasergebilde ist eine Plasmabehandlung, um insbesondere die Hydrophilie der Fasern oder Fasergebilde zu erhöhen. Plasma ist ein Gemisch aus neutralen und geladenen Teilchen. In speziellen Fällen liegen nur geladene Teilchen vor. Im Plasma liegen verschieden Spezies wie Elektronen, Kationen, Anionen, neutrale Atome, neutrale oder geladene Moleküle vor. Durch die im Plasma enthaltenen aktiven Teilchen können Oberflächen wie z.B. Fasern oder Vliesstoffe modifiziert werden. Dabei können verschiedene Effekte erzielt werden wie z.B. eine Veränderung der Oberfläche durch Plasmaätzen, Plasmaaktivierung oder Plasmapolymerisation. Bei der Plasmaaktivierung wird die Oberfläche durch ein Plasma unter Zusatz von Sauerstoff aktiviert. Bei der Plasmapolymerisation werden weitere organische Vorläuferverbindungen in die Prozesskammer gegeben.

Die Fasern oder Faserhilfsstoffe können durch das Tempern hydrophob ausgebildet werden, da durch das Tempern die Faserhilfsmittel und Faserverarbeitungsmittel reduziert werden können. Die Plasmabehandlung kann sowohl unter Atmosphärendruck als auch unter Vakuum insbesondere unter Zusatz von Sauerstoff durchgeführt werden. Auch können weitere Substanzen wie Acrylsäure während der Plasmabehandlung zugeben werden.

Zudem ist eine bevorzugte Nachbehandlung die Sterilisation der Fasern oder Fasergebilde für die Anwendung insbesondere für Wundauflagen. Bevorzugt wird die Sterilisation durch Strahlensterilisation oder durch Sterilisation mit Ethylenoxid durchgeführt. Durch die Sterilisation können die Eigenschaften wie z.B. Absorptionskapazität und/oder Höchstzugkraft und Höchstzugkraftdehnung im hydrogelierten Zustand positiv beeinflusst werden.

Die einzelnen Verfahrensschritte, Tempern, Verfestigen, Zumischen von weiteren Fasern, Zugabe von Additiven, Zugabe von Verarbeitungshilfsstoffen, und Nachbehandeln können mehrfach in beliebiger Reihenfolge wiederholt werden. Als zweckmäßig hat sich erwiesen die Fasern oder die Fasergebilde zumindest einmal dabei bei einer vorbestimmten Tempertemperatur eine vorbestimmte Temperdauer lang zu tempern.

Als Verarbeitungshilfsstoffe können Avivage, antistatische Mittel, Tenside, Stabilisatoren, Gleitmittel oder dergleichen zum Einsatz kommen.

In einer bevorzugten Variante des Herstellungsverfahrens werden die Fasern aus einem ersten Faserrohmaterial, insbesondere wasserlösliche Polyvinylalkohol-Stapelfasern, zur Vernetzung bei einer vorbestimmten Tempertemperatur, die oberhalb der Glasübergangstemperatur und unterhalb der Schmelztemperatur der Fasern aus einem ersten Faserrohmaterial liegt, insbesondere 10 min bis 7h lang, getempert. Darauffolgend kann optional eine Zumischung von weiteren Fasern, insbesondere von nicht-gelierenden Fasern, besonders bevorzugt von Polyester-Fasern, mit einem Gewichts-Anteil von 10 bis 50 Gew.% vorgenommen werden. Dann kann aus den so hergestellten Fasern ein zweidimensionales Fasergebilde, wie zum Beispiel ein Vliesstoff, gegebenenfalls unter Einsatz von Verarbeitungshilfsstoffen, wie zum Beispiel Avivage oder antistatischen Mitteln, mittels eines Verfestigungsverfahrens hergestellt werden.

In einer anderen bevorzugten Variante des Herstellungsverfahrens können optional Fasern aus einem ersten Faserrohmaterial mit weiteren Fasern aus einem zweiten Faserrohmaterial vermischt werden, wobei der Anteil an weiteren Fasern bevorzugt 10-50 Gew.% beträgt. Es können aber auch nur Fasern aus einem ersten Faserrohmaterial verwendet werden. Bevorzugt werden als Fasern aus einem ersten Faserrohmaterial Polyvinylalkohol-Fasern und als weitere Fasern aus einem zweiten Faserrohmaterial Polyester-Fasern verwendet. Aus diesen Fasern kann mittels eines Verfestigungsverfahrens ein zweidimensionales Fasergebilde wie zum Beispiel ein Vliesstoff hergestellt werden. Nachfolgend kann das so hergestellte zweidimensionale Fasergebilde bei einer Tempertemperatur oberhalb der Glasübergangstemperatur und unterhalb der Schmelztemperatur der Fasern aus einem ersten Faserrohmaterial getempert werden. Optional kann ein derartig hergestelltes zweidimensionales Fasergebilde nachbehandelt werden.

In einem weiteren Aspekt der Erfindung wird die Verwendung von Fasergebilden, wie vorhergehend beschrieben, vorgeschlagen, wobei derartige Fasergebilde insbesondere zur Herstellung von Materialien für medizinische Anwendungen, insbesondere für Wundauflagen und Wundverbände angewendet wird und insbesondere für die Herstellung von Wundauflagen für den Bereich der modernen Wundversorgung.

Zudem können die Fasergebilde zur Herstellung von anderen Materialien für medizinische Anwendungen wie Nahtmaterialien, Implantate, Tissue Egineering Scaffolds, transdermale Patches, Drug-delivery Produkte, Trägermaterialien oder Ostomieprodukte angewendet werden.

Auch ist eine Verwendung zur Herstellung von Trägermaterialien, Dämmstoffen, Filtermaterialien zur Herstellung von Hygiene-, Kosmetik,-Haushaltsprodukten, technischen Absorberprodukten, wie Kabelummantelungen, Produkten für den Lebensmittelbereich, wie Lebensmittelverpackungen möglich. Als Hygieneprodukte können unter anderem Damenhygieneprodukte, Windeln und Inkontinenzprodukte verstanden werden. Von den Haushaltsprodukten werden ebenfalls Reinigungsmaterialien umfasst.

Für die jeweilige Verwendung ergeben sich unter anderem die vorhergehend genannten Vorteile.

Als weiterer Aspekt der Erfindung wird ein Wundverband oder eine Wundauflage, enthaltend Fasergebilde wie vorhergehend beschrieben, vorgeschlagen. Derartige Fasergebilde können bevorzugt im Bereich der Modernen Wundversorgung, insbesondere zur modernen (feuchten) Wundbehandlung, eingesetzt werden.

Bei der modernen Wundversorgung stellen die Wundauflagen ein optimales feuchtes Wundklima ein, durch das die Wunde schneller abheilen kann. Die moderne Wundversorgung wird zur Behandlung von schwer heilenden Wunden wie chronischen Wunden verwendet, die z.B. durch Druckbeanspruchung oder Wundliegen (Dekubitus), Diabetes, Durchblutungsstörungen, Stoffwechselerkrankungen, Gefäßerkrankungen wie Veneninsuffizienz oder Immunschwäche entstehen können.

Die Fasern oder erfindungsgemäßen Fasergebilde besitzen zum einem eine hohe Absorptionskapazität für wässrige Lösungen und können somit das Wundexsudat absorbieren und einschließen. Durch die Aufnahme des Wundexsudats bilden die Fasern oder Fasergebilde zum anderen ein Hydrogel aus, welches die Flüssigkeit fest einschließt und auch unter Druck, der z.B. durch Anlegen eines Verbandes entsteht, zurückhält. Durch die Ausbildung des Hydrogels wird zudem ein feuchtes Wundklima geschaffen, durch das die Wundheilung gefördert wird. Die hydrogelierten Fasern oder Fasergebilde passen sich an die Struktur der Wundoberfläche an und können insbesondere auch für die Behandlung von Wundhöhlen verwendet werden. Durch die hohe Höchstzugkraft und Höchstzugkraftdehnung können die hydrogelierten Fasern oder Fasergebilde problemlos in einem Stück von der Wunde oder aus der Wundhöhle entfernt werden, ohne diese zu verletzen.

Solche Wundverbände oder Wundauflagen können auch analog klassischer Wundverbände oder Wundauflagen, wie beispielsweise Mullbinden, angewendet werden, weisen aber die vorteilhaften hydrogelierenden Eigenschaften auf, so dass durch erfindungsgemäße Wundverbände oder Wundauflagen ein vorteilhaft verbesserte Wundversorgung erreicht werden kann.

Ausführung der Erfindung

**Methoden und Messmethoden**

Im Folgenden wird dargelegt, wie verschiedene zur Charakterisierung der Fasern oder der erfindungsgemäßen Fasergebilde verwendbare Parameter erfindungsgemäß zu bestimmen sind:

**1) Bestimmung der Dicke der zweidimensionalen Fasergebilde und / oder Vliesstoffs**

Gemäß DIN EN ISO 9073-2, jedoch ohne Konditionierung

**2) Bestimmung des Flächengewichtes der zweidimensionalen Fasergebilde und / oder Vliesstoffs**

Gemäß DIN EN 29073, jedoch ohne Konditionierung

**3) Bestimmung der Absorptionskapazität von Fasern**

Ein 600 mL Becherglas wird mit 300 mL 0,9%iger Natriumchlorid-Lösung (0,9 g Natriumchlorid in 100 mL destilliertem Wasser gelöst) oder mit einer Lösung gemäß der in DIN 13726-1 in Punkt 3.2.2.3 aufgeführten Prüflösung A gefüllt. Es werden 0,40 g (Fasergewicht trocken: $m_{trocken}$) der Fasern in die Lösung eingerührt. Die Fasern bleiben 10 min unter gelegentlichen Rühren mittels Glasstab im Becherglas. Die Zeiterfassung erfolgt mit einer Stoppuhr. Ein vortariertes Metallsieb (32 mesh) wird auf ein 2000 mL Becherglas gelegt. Der gesamte Inhalt des 600 mL Becherglases wird über das Metallsieb gegossen. Die Fasern werden 5 min im Metallsieb abgetropft. Das Gewicht des Metallsiebes einschließlich der Fasern wird ermittelt. Das Tara des Metallsiebes wird vom Gewicht abgezogen. Man erhält das Fasergewicht der hydrogelierten Fasern ($m_{nass}$).

Die Ermittlung der Absorptionskapazität der Fasern erfolgt mit folgender Formel:

$$\text{Relative } Absorptionskapazität\left[{}^{g}\!/_{g}\right] = \frac{m_{nass} - m_{trocken}}{m_{trocken}}$$

Dabei ist

$m_{nass}$ die Masse aus der Messprobe und der absorbierten Flüssigkeit am Ende der Prüfung in g
$m_{trocken}$ die Masse der trockenen Messprobe in g

4) **Bestimmung der Absorptionskapazität von zweidimensionalen Fasergebilden oder Vliesstoffen in Anlehnung an DIN EN ISO 9073-6**

Die Prüfung der Absorptionskapazität erfolgt in Anlehnung an DIN EN ISO 9073-6; Absorption von Flüssigkeiten.
Als vorgegebene Flüssigkeit (Prüfmedium) laut Punkt 5.2.7 in der DIN EN ISO 9073-6 wird eine 0,9%ige Natriumchlorid-Lösung (0,9 g Natriumchlorid in 100 mL destilliertem Wasser) oder eine Prüflösung A gemäß der DIN 13726-1 Punkt 3.2.2.3 verwendet.
Das verwendete Prüfmedium wird bei dem jeweiligen Messergebnis mit angegeben.
Das Vorbereiten der Messproben (Größe von 10*10 cm) und Durchführung der Bestimmung erfolgt analog der DIN EN ISO 9073-6, jedoch ohne Konditionierung.
Die Absorptionskapazität wurde zudem abweichend von der Norm nach zwei verschiedenen Absorptionszeiten bestimmt:

1) Absorptionskapazität nach 1 min: gemäß Norm werden die Messproben 1 min in das Prüfmedium getaucht und 2 min abtropfen gelassen
2) Absorptionskapazität nach 1h: die Messproben werden 1h in das Prüfmedium getaucht und 2 min abtropfen gelassen

Die Absorption von Flüssigkeit (LAC) in Prozent berechnet sich laut DIN EN ISO 9073-6 nach folgender Formel:

$$LAC\left[\%\right] = \frac{m_n - m_k}{m_k} \times 100$$

Dabei ist
$m_k$ die Masse der trockenen Messprobe in g
$m_n$ die Masse aus der Messprobe und der absorbierten Flüssigkeit am Ende der Prüfung in g
Die relative Absorption in g/g errechnet sich wie folgt:

$$Relative\ Absorption\left[{}^{g}\!/_{g}\right] = \frac{m_n - m_k}{m_k}$$

Die absolute Absorption in g/m$^2$ errechnet sich wie folgt:

$$Absolute\ Absorption\left[{}^{g}\!/_{m^2}\right] = Relative\ Absorption\left[{}^{g}\!/_{g}\right] \times Flächengewicht\left[g/m^2\right]$$

Die hydrogelierten Messproben nach der Bestimmung der Absorptionskapazität nach 1h werden weiterverwendet für die Bestimmung des Rückhaltvermögens (Retention) von zweidimensionalen Fasergebilden und / oder Vliesstoffen (Punkt 5) und Bestimmung des löslichen Anteils von zweidimensionalen Fasergebilde und / oder Vliesstoffe (Punkt 6).

**5) Bestimmung des Rückhaltvermögens (Retention) von zweidimensionalen Fasergebilden oder Vliesstoffen**

Für die Bestimmung werden die hydrogelierten Messproben nach der Bestimmung der Absorptionskapazität (Punkt 4) nach 1h verwendet (Absorptionskapazität nach 1h); zudem werden die ermittelten Werte der Massen von den trockenen Messproben, die bei der Bestimmung der Absorptionskapazität ermittelt wurden, verwendet:
$m_k$ die Masse der trockenen Messprobe in g
Die Messproben werden jeweils auf einen flachen Metallnetz mit einer Größe von 15 x 15 cm gelegt, welches über einer Schüssel platziert ist, so dass Flüssigkeit von der Messprobe in die Schüssel ablaufen kann.
Die Messprobe wird mit einem Gewicht, welches flächig einen Druck von 40 mmHg auf die gesamte Fläche der Messprobe ausübt (dies entspricht einem Gewicht von 5,434 kg auf einer Fläche von 100 cm$^2$) über einen Zeitraum von 2 min beaufschlagt. Danach wird das Gewicht der Messprobe genau abgewogen ($m_{Druck}$)
Das relative Rückhaltevermögen (Retention) in g/g errechnet sich wie folgt:

$$Relatives\ R\ddot{u}ckhaltevermögen\ (Retention)\left[\frac{g}{g}\right] = \frac{m_{Druck} - m_k}{m_k}$$

Das Rückhaltevermögen (Retention) in Prozent errechnet sich wie folgt:

$$R\ddot{u}ckhaltevermögen\ (Retention)[\%] = \frac{Relatives\ R\ddot{u}ckhaltevermögen}{Relative\ Absorption\ nach\ 1h} * 100$$

**6) Bestimmung des löslichen Anteils von zweidimensionalen Fasergebilden oder Vliesstoffen**

Für die Bestimmung werden die hydrogelierten Messproben nach der Bestimmung der Absorptionskapazität (Punkt 4) nach 1h verwendet (Absorptionskapazität nach 1h); zudem werden die ermittelten Werte der Massen von den trockenen Messproben, die bei der Bestimmung der Absorptionskapazität ermittelt wurden, verwendet:
$m_k$ die Masse der trockenen Messprobe in g
Die hydrogelierte Messprobe wird in ein tariertes 100 mL Becherglas ($m_{Becherglas}$) gelegt. Das Becherglas mit Messprobe wird in einen handelsüblichen Labortrockenschrank mit Umluft mit einer Temperatur von 70°C gestellt und die hydrogelierte Messprobe dadurch getrocknet. Nach 24h wird das Becherglas mit der getrockneten Messprobe aus dem Trockenschrank heraus genommen. Nach Abkühlen wird das Gewicht der Messprobe ($m_{trocken}$) bestimmt, wobei das Becherglas zusammen mit der Messprobe ($m_{gesamt}$) ausgewogen wird und das Gewicht des Becherglases von dem Gewicht abgezogen wird:

$$m_{trocken} = m_{gesamt} - m_{Becherglas}$$

Der lösliche Anteil in Prozent wird wie folgt berechnet:

$$L\ddot{o}slicher\ Anteil\ [\%] = 100 - \left(\frac{m_{trocken}}{m_k} * 100\right)$$

**7) Bestimmung des Schrumpfes von zweidimensionalen Fasergebilden oder Vliesstoffen**

Der Schrumpf wird durch Ausstanzen von 10,0 cm x 10,0 cm (Fläche1) großen Stücken und Eintauchen derselben in ein Prüfmedium bestimmt. Das Prüfmedium ist entweder eine 0,9 %ige wässrige Natriumchlorid-Lösung oder in eine Prüflösung A gemäß DIN 13726-1 Punkt 3.2.2.3. Das jeweilige Prüfmedium wird bei dem Messergebnis mit angegeben.
Die ausgestanzten und getränkten Stücke werden nach 1h aus der Lösung entnommen und für 2min abgetropft. Danach wird die Größe der Stücke abgemessen (Fläche 2). Der Schrumpf der Vliesstoffe kann dann nach folgender Formel berechnet werden:

$$Schrumpf\ [\%] = 100 - \left(\frac{Fläche2\ [cm^2]}{Fläche1\ [cm^2]}\right) * 100$$

**8) Bestimmung der Höchstzugkraft und der Höchstzugkraftdehnung bei maximaler Zugkraft von zweidimensionalen Fasergebilden und / oder Vliesstoffen im hydrogeliertem Zustand**

Für die Bestimmung werden DIN A4 große Vliesstoffstücke ausgestanzt und in einen Überschuss von 0,9%iger Natriumchlorid-Lösung oder Prüflösung A gemäß DIN 13726-1 Punkt 3.2.2.3 gelegt. Die Vliesstoffstücke werden nach 1h aus der Lösung entnommen. Mit Hilfe eines Stanzeisen werden aus den hydrogelierten Vliesstoff-Stücken die Messproben sowohl in Längsrichtung (Maschinenrichtung) des Vliesstoffes als auch in Querrichtung des Vliesstoffes gestanzt.

Das Stanzeisen zum Ausstanzen der Messprobe hat eine Länge von 90 mm. Die Breite beträgt am oberen und unteren Ende 35 mm. Nach 20 mm verengt sich das Stanzeisen jeweils an beiden Enden auf 20 mm (siehe Bild 1). Die Bestimmung der Höchstzugkraft und Höchstzugkraftdehnung erfolgt dann gemäß EN 29073-03 mit einer Zwick Z 1.0, jedoch mit folgenden Abweichungen

- Keine Konditionierung
- Abzugsgeschwindigkeit 200 mm/min
- Anderes Stanzeisen (wie oben beschrieben); Einspannlänge angepasst an die Stanzeisenlänge
- Andere Probenvorbereitung: die Proben werden nicht im trockenen Zustand, sondern im hydrogeliertem Zustand vermessen (Erstellung der Messproben wie oben beschrieben)

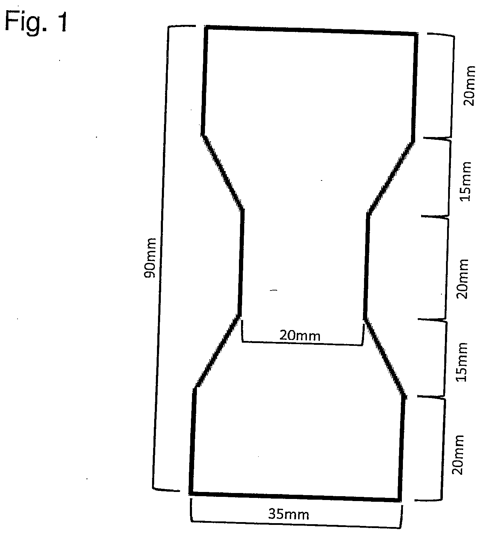

In Fig 1 ist das Stanzeisen zum Ausstanzen der Messproben dargestellt

**9) Bestimmung der Löslichkeit von wasserlöslichen Fasern**

Ein 250 mL Becherglas wird mit 200 mL destilliertem Wasser gefüllt und mit einer Heizplatte auf Prüftemperatur (Temperatur, bei der die Fasern aus Polyvinylalkohol wasserlöslich sind). Temperaturkontrolle erfolgt durch ein Thermometer.

Es werden jeweils 0,4 g der Fasern in die 200 mL des temperierten Wassers kurz eingerührt. Die Fasern bleiben zunächst 3 min ohne Rühren im Becherglas. Danach wird der Becherglasinhalt 7 min kräftig gerührt. Die Zeiterfassung erfolgt jeweils mit der Stoppuhr. Abschließend erfolgt eine optische Prüfung (mit dem Auge), ob sich die Fasern vollständig gelöst haben. Eine 100 prozentige Wasserlöslichkeit liegt dann vor, wenn keine festen Fasern oder Faserbestandteile mehr in der Lösung sichtbar sind.

**10) Bestimmung der Thermodesorption**

Bei der Bestimmung der Thermodesorption werden durch Aufheizen einer Probe von Fasern oder Fasergebilden bei 150°C für 20 min in den Fasern enthaltene organische Komponenten freigesetzt, mittels Kryotrap fokussiert und danach mittels Kaltaufgabesystem in die GC/MS injiziert. Dabei wird ein Thermodesorptionssystem GERSTEL und ein Kaltaufgabesystem KAS GERSTEL verwendet. Mittels GC/MS werden die freigesetzten Komponenten detektiert. Dabei wird ein GC Agilent Technologies 6890N Network GC System, Massenselektiver Detektor Agilent Technologies 5973 verwendet.

**11) Bestimmung der Netzzeit von zweidimensionalen Fasergebilden oder Vliesstoffen**

Es wird die Zeitdauer gemessen, die 1 Tropfen destilliertes Wasser braucht, um in das Fasergebilde oder den Vliesstoff einzusinken. Die Prüfung wird insgesamt mit 5 Tropfen durchgeführt und der Mittelwert gebildet.

**12) Untersuchung der Fasern oder Fasergebilde mittels XPS**

Die Messungen mittels XPS (X-ray Photoelectron Spectroscopy) wurden mit einem SSX-100 Spektrometer (Fa. SSI, US) mit monoenergetischer Al K$\alpha$1,2 Anregung (1486,6 eV) im Ultrahochvakuum (10-9 Torr) durchgeführt. Die Informationstiefe liegt zwischen 6 und 10 nm. Die Ladungskompensation für nichtleitende Proben wird mittels Floodgun erzielt. Vor Messbeginn werden die Proben über Nacht vakuumgelagert.

**Beispiele**

**Beispiel 1: Herstellung von getemperten Fasern aus wasserlöslichen Polyvinylalkohol**

Wasserlösliche Stapelfasern aus Polyvinylalkohol (2,2 dtex, 51 mm) werden im Faserballenöffner geöffnet. Die Stapelfasern aus Polyvinylalkohol sind bei einer Temperatur unterhalb von 25°C wasserlöslich. Nach dem Öffnen des Faserballens werden die Fasern bei 150°C getempert (z.B. in einem handelsüblichen Labortrockenschrank mit Umluft), um eine Vernetzung des Polyvinylalkohols zu erzielen. Ab einer Temperdauer von 2h stellt sich eine Stabilität der PVA-Fasern ein, die sich durch die Ausbildung von stabilen, hydrogelierenden Fasern in 0,9 %iger wässriger Natriumchlorid-Lösung oder in Prüflösung A gemäß DIN 13726-1 Punkt 3.2.2.3 zeigt. Die Stabilität der Fasern steigt mit der Temperdauer. Bei einer Temperdauer von 4 bis 7h weisen die Fasern eine hohe Stabilität auf.

Nach dem Tempern wird die Absorption von 0,9%iger wässriger Natriumchlorid-Lösung der Fasern bestimmt. Die Bestimmung der Absorption erfolgt in wie in Messmethoden Punkt 3 beschrieben (Bestimmung der Absorptionskapazität von Fasern). Die relative Absorptionskapazität mit 0,9 %iger wässriger Natriumchlorid-Lösung als Prüfmedium beträgt abhängig von der Temperdauer und damit des Vernetzungsgrads zwischen 3 bis 40 g/g. Die getemperten Fasern aus Polyvinylalkohol können zu Vliesstoffen weiterverarbeitet werden. Es werden Vliesstoffe aus Polyvinylalkohol-Fasern oder aus Polyvinylalkohol-Fasern mit Zumischung anderer Fasern wie z.B. Polyester hergestellt. Die aus den getemperten PVA-Fasern hergestellten Vliesstoffe zeigen abhängig von der Faserzumischung und dem Vernetzungsgrad eine hohe relative Absorptionskapazität mit 0,9%iger wässriger Natriumchlorid-Lösung als Prüfmedium zwischen 4 bis 35 g/g.

**Beispiel 2: Nadelverfestigte Vliesstoffe aus wasserlöslichen Polyvinylalkohol-Fasern mit anschließender thermischer Vernetzung**

Es wird ein nadelverfestigter Vliesstoff aus wasserlöslichen Polyvinylalkohol-Stapelfasern hergestellt. Die Polyvinylalkohol-Fasern sind bei einer Temperatur unterhalb von 25°C wasserlöslich und besitzen einen Fasertiter von 1,7 oder 2,2 dtex bei einer Stapelfaserlänge von 38 bzw. 51 mm. Die Polyvinylalkohol-Fasern werden durch eine Krempel zu einem Vlies gelegt und anschließend durch Vernadelung mit einer Einstichdichte von 100-170 Einstichen pro Quadratcentimeter vernadelt. Die nadelverfestigten Polyvinylalkohol-Vliesstoffe werden bei einer Temperatur von 150°C getempert, um eine Stabilisierung des Polyivnylalkohols zu erzielen. Die Vliesstoffe werden dabei in einem handelsüblichen Labortrockenschrank mit Umluft getempert. Ab einer Temperdauer von 2h stellt sich eine Stabilität der Polyvinylalkohol-Vliesstoffe ein, die sich durch die Ausbildung von stabilen, hydrogelierenden Vliesen in 0,9 %iger wässriger Natriumchlorid-Lösung oder Prüflösung A gemäß DIN 13726-1 Punkt 3.2.2.3 zeigt. Die Stabilität der Vliesstoffe steigt mit der Temperdauer. Bei einer Temperdauer von 2,5 bis 7h weisen die Vliesstoffe eine hohe Stabilität auf. Der lösliche Anteil der Vliesstoffe liegt nach 1h in Prüflösung A bei maximal 20%. Nach dem Tempern wird die relative Absorptionskapazität nach 1 min und 1h mit Prüflösung A als Prüfmedium bestimmt. Die relative Absorptionskapazität nach 1 min beträgt zwischen 5 und 20 g/g. Die relative Absorptionskapazität nach 1 h beträgt zwischen 5 und 20 g/g. Weiterhin wurde das Rückhaltevermögen (Retention) der Vliesstoffe nach 1h in Prüflösung A bestimmt. Diese beträgt zwischen 80-100%. Zudem wird der Schrumpf der verfestigten Vliesstoffe in Prüflösung A nach 1h in Prüflösung A bestimmt. Der Schrumpf der Polyvinylalkohol-Vliesstoffe beträgt abhängig von der Temperdauer und damit dem Vernetzungsgrad der Vliesstoffe zwischen 30 und 60%.

Tabelle 1: Beispiel für einen nadelverfestigten Vliesstoff aus wasserlöslichen Polyvinylalkohol-Fasern, der getempert wurde

| Parameter | Beschreibung / Ergebnis |
|---|---|
| Polyvinylalkohol-Fasern | 1,5 bis 2,2 dtex, 40-70 mm |
| Temperatur, bei der die Polyvinylalkohol-Fasern wasserlöslich sind | Unterhalb von 25°C |
| Anteil an Polyvinylalkohol-Fasern [%] | 100 |
| Temperdauer bei 150°C [min] | 150-300 |
| Verfestigungsart | Vernadelung |
| Einstichdichte [#/cm$^2$] | 100-170 |
| Flächengewicht [g/m$^2$] | 150-210 |
| Dicke [mm] | 1,5-3,0 |

(fortgesetzt)

| Parameter | Beschreibung / Ergebnis |
|---|---|
| Relative Absorptionskapazität [g/g] nach 1 min in Prüflösung A | 5,0-20,0 |
| Relative Absorptionskapazität [g/g] nach 1h in Prüflösung A | 5,0-20,0 |
| Rückhaltevermögen (Retention) [%] nach 1h in Prüflösung A | 80-100 |
| Löslicher Anteil nach 1h in Prüflösung A [%] | 0-20 |
| Schrumpf [%] | 30-50 |
| Höchstzugkraft im hydrogeliertem Zustand [N/2cm]; längs | 1-20 |
| Höchstzugkraftdehnung im hydrogeliertem Zustand [%]; längs | 80-300 |
| Höchstzugkraft im hydrogeliertem Zustand [N/2cm]; quer | 1-20 |
| Höchstzugkraftdehnung im hydrogeliertem Zustand [%]; quer | 80-300 |

**Beispiel 3: Kalander-verfestigte Vliesstoffe aus wasserlöslichen Polyvinylalkohol-Fasern mit anschließender thermischer Vernetzung**

Es wird ein Kalander-verfestigter Vliesstoff aus wasserlöslichen Polyvinylalkohol-Stapelfasern hergestellt. Die Polyvinylalkohol-Fasern sind bei einer Temperatur unterhalb von 25°C wasserlöslich und besitzen einen Fasertiter von 2,2 dtex bei einer Stapelfaserlänge 51 mm. Die Polyvinylalkohol-Fasern werden durch eine Krempel zu einem Vlies gelegt und anschließend durch thermische Verfestigung mittels eines Kalanders mit PS (point seal)-Gravur verfestigt. Die thermisch verfestigten Polyvinylalkohol-Vliesstoffe werden bei einer Temperatur von 150°C getempert, um eine Stabilisierung des Polyivnylalkohols zu erzielen. Die Vliesstoffe werden dabei in einem handelsüblichen Labortrockenschrank mit Umluft getempert. Ab einer Temperdauer von 2h stellt sich eine Stabilität der Polyvinylalkohol-Vliesstoffe ein, die sich durch die Ausbildung von stabilen, hydrogelierenden Vliesen in 0,9 %iger wässriger Natriumchlorid-Lösung oder Prüflösung A gemäß DIN 13726-1 Punkt 3.2.2.3 zeigt. Die Stabilität der Vliesstoffe steigt mit der Temperdauer. Bei einer Temperdauer von 2,5 bis 7h weisen die Vliesstoffe eine hohe Stabilität auf. Der lösliche Anteil der Vliesstoffe liegt nach 1h in Prüflösung A bei maximal 20%. Nach dem Tempern wird die relative Absorptionskapazität nach 1 min und 1h mit Prüflösung A als Prüfmedium bestimmt. Die relative Absorptionskapazität nach 1 min beträgt zwischen 5 und 20 g/g. Die relative Absorptionskapazität nach 1 h beträgt zwischen 5 und 20 g/g. Weiterhin wurde das Rückhaltevermögen (Retention) der Vliesstoffe nach 1h in Prüflösung A bestimmt. Diese beträgt zwischen 80-100%. Zudem wird der Schrumpf der verfestigten Vliesstoffe in Prüflösung A nach 1h in Prüflösung A bestimmt. Der Schrumpf der Polyvinylalkohol-Vliesstoffe beträgt abhängig von der Temperdauer und damit dem Vernetzungsgrad der Vliesstoffe zwischen 30 und 60%.

Tabelle 2: Beispiel für einen thermisch verfestigten Vliesstoff aus wasserlöslichen Polyvinylalkohol-Fasern, der getempert wurde

| Parameter | Beschreibung / Ergebnis |
|---|---|
| Polyvinylalkohol-Fasern | 2,2 dtex, 51 mm |
| Temperatur, bei der die Polyvinylalkohol-Fasern wasserlöslich sind | Unterhalb von 25°C |
| Anteil an Polyvinylalkohol-Fasern [%] | 100 |
| Temperdauer bei 150°C [min] | 150-300 |
| Verfestigungsart | Thermisch mit Kalander (PS-Gravur) |
| Flächengewicht [g/m$^2$] | 150-210 |
| Dicke [mm] | 0,8-3,0 |
| Relative Absorptionskapazität [g/g] nach 1 min in Prüflösung A | 5,0-20,0 |
| Relative Absorptionskapazität [g/g] nach 1h in Prüflösung A | 5,0-20,0 |
| Rückhaltevermögen (Retention) [%] nach 1h in Prüflösung A | 80-100 |

(fortgesetzt)

| Parameter | Beschreibung / Ergebnis |
|---|---|
| Löslicher Anteil nach 1h in Prüflösung A [%] | 0-20 |
| Schrumpf [%] | 20-50 |
| Höchstzugkraft im hydrogeliertem Zustand [N/2cm]; längs | 2-30 |
| Höchstzugkraftdehnung im hydrogeliertem Zustand [%]; längs | 100-400 |
| Höchstzugkraft im hydrogeliertem Zustand [N/2cm]; quer | 2-30 |
| Höchstzugkraftdehnung im hydrogeliertem Zustand [%]; quer | 100-400 |

**Beispiel 4: Mischvliesstoffe aus wasserlöslichen Polyvinylalkohol-Fasern und Polyester-Fasern mit anschließender thermischer Vernetzung**

Es werden nadelverfestigte Mischvliesstoffe aus wasserlöslichen Polvinylalkohol-Stapelfasern (2,2 dtex) und Polyester-Stapelfasern hergestellt. Der Anteil der Polyesterfasern im Mischvliesstoff beträgt zwischen 10 und 50%.

Die Polyvinylalkohol-Fasern sind bei einer Temperatur unterhalb von 25°C wasserlöslich und besitzen einen Fasertiter von 2,2 dtex bei einer Stapelfaserlänge 51 mm. Die Polyester-Fasern besitzen einen Fasertiter von 1,7 dtex bzw. 3,3 dtex und eine Stapelfaserlänge von 38 mm bzw. 51 mm. Die Vliesstoffe werden bei 150°C getempert, um eine Vernetzung der Polyvinylalkohol-Fasern im Vlies zu erzielen. Die Vliesstoffe werden dabei in einem handelsüblichen Labortrockenschrank mit Umluft getempert Ab einer Temperdauer von 2h stellt sich eine Stabilität der Polyvinylalkohol-Vliesstoffe ein, die sich durch die Ausbildung von stabilen, hydrogelierenden Vliesen in 0,9 %iger wässriger Natriumchlorid-Lösung oder Prüflösung A gemäß DIN 13726-1 Punkt 3.2.2.3 zeigt. Die Stabilität der Vliesstoffe steigt mit der Temperdauer. Bei einer Temperdauer von 2,5 bis 7h weisen die Vliesstoffe eine hohe Stabilität auf. Der lösliche Anteil der Vliesstoffe liegt nach 1h in Prüflösung A bei maximal 20%. Nach dem Tempern wird die relative Absorptionskapazität nach 1 min und 1h mit Prüflösung A als Prüfmedium bestimmt. Die relative Absorptionskapazität nach 1 min beträgt zwischen 7 und 25 g/g. Die relative Absorptionskapazität nach 1 h beträgt zwischen 7 und 25 g/g. Weiterhin wurde das Rückhaltevermögen (Retention) der Vliesstoffe nach 1h in Prüflösung A bestimmt. Diese beträgt zwischen 80-100%. Zudem wird der Schrumpf der verfestigten Vliesstoffe in Prüflösung A nach 1h in Prüflösung A bestimmt. Der Schrumpf der Polyvinylalkohol-Vliesstoffe beträgt abhängig von der Temperdauer und damit dem Vernetzungsgrad der Vliesstoffe zwischen 1 und 45%. Der Schrumpf der Mischvliesstoffe ist somit deutlich niedriger im Vergleich zu Polyvinylalkohol-Vliesstoffen ohne Polyesterfaser-Zumischung. Mit steigendem Anteil an Polyester im Vliesstoff erniedrigt sich der Schrumpf.

Tabelle 3: Beispiel für einen nadelverfestigten Vliesstoff aus wasserlöslichen Polyvinylalkohol-Fasern mit Zumischung von Polyester-Fasern, der getempert wurde.

| Parameter | Beschreibung / Ergebnis |
|---|---|
| Polyvinylalkohol-Fasern | 2,2 dtex, 51 mm |
| Temperatur, bei der die Polyvinylalkohol-Fasern wasserlöslich sind | Unterhalb von 25°C |
| Anteil an Polyvinylalkohol-Fasern [%] | 50 bis 100 |
| Polyester-Fasern | 1,7 dtex bzw. 3,3 dtex, 38 mm bzw. 51 |
| Anteil an Polyester-Fasern [%] | 0 bis 50 |
| Temperdauer bei 150°C [min] | 150-300 |
| Verfestigungsart | Mechanisch durch Vernadelung |
| Einstichdichte [#/cm$^2$] | 100-170 |
| Flächengewicht [g/m$^2$] | 150-210 |
| Dicke [mm] | 0,8-3,0 |

(fortgesetzt)

| Parameter | Beschreibung / Ergebnis |
|---|---|
| Relative Absorptionskapazität [g/g] nach 1 min in Prüflösung A | 7,0-25,0 |
| Relative Absorptionskapazität [g/g] nach 1h in Prüflösung A | 7,0-25,0 |
| Rückhaltevermögen (Retention) [%] nach 1h in Prüflösung A | 80-100 |
| Löslicher Anteil nach 1h in Prüflösung A [%] | 0-30 |
| Schrumpf [%] | 1 bis 45 % |
| Höchstzugkraft im hydrogeliertem Zustand [N/2cm]; längs | 4-30 |
| Höchstzugkraftdehnung im hydrogeliertem Zustand [%]; längs | 100-400 |
| Höchstzugkraft im hydrogeliertem Zustand [N/2cm]; quer | 4-30 |
| Höchstzugkraftdehnung im hydrogeliertem Zustand [%]; quer | 100-400 |

**Beispiel 5: Mischvliesstoffe aus wasserlöslichen Polyvinylalkohol-Fasern und Polyvinylalkohol-Faser, die ab einer Temperatur von 70°C wasserlöslich sind mit anschließender thermischer Vernetzung**

Es werden nadelverfestigte Mischvliesstoffe aus wasserlöslichen Polvinylalkohol-Stapelfasern (2,2 dtex) (unterhalb von 25°C wasserlöslich) und Polyvinylalkohol-Stapelfasern, die ab einer Temperatur von 70°C wasserlöslich sind, hergestellt. Der Anteil der Polyvinylalkohol-Faser, die ab einer Temperatur von 70°C wasserlöslich sind, beträgt 20 und 35%. Die wasserlöslichen Polyvinylalkohol-Fasern sind bei einer Temperatur unterhalb von 25°C wasserlöslich und besitzen einen Fasertiter von 2,2 dtex bei einer Stapelfaserlänge 51 mm. Die Polyvinylalkohol-Fasern, die ab einer Temperatur von 70°C wasserlöslich sind besitzen einen Fasertiter von 1,7 dtex und eine Stapelfaserlänge von 38 mm. Die Vliesstoffe werden bei 150°C getempert, um eine Vernetzung der wasserlöslichenvPolyvinylalkohol-Fasern im Vlies zu erzielen. Die Vliesstoffe werden dabei in einem handelsüblichen Labortrockenschrank mit Umluft getempert. Ab einer Temperdauer von 2h stellt sich eine Stabilität der Polyvinylalkohol-Vliesstoffe ein, die sich durch die Ausbildung von stabilen, hydrogelierenden Vliesen in 0,9 %iger wässriger Natriumchlorid-Lösung oder Prüflösung A gemäß DIN 13726-1 Punkt 3.2.2.3 zeigt. Die Stabilität der Vliesstoffe steigt mit der Temperdauer. Bei einer Temperdauer von 2,5 bis 7h weisen die Vliesstoffe eine hohe Stabilität auf. Der lösliche Anteil der Vliesstoffe liegt nach 1h in Prüflösung A bei maximal 20%. Nach dem Tempern wird die relative Absorptionskapazität nach 1 min und 1h mit Prüflösung A als Prüfmedium bestimmt. Die relative Absorptionskapazität nach 1 min beträgt zwischen 7 und 25 g/g. Die relative Absorptionskapazität nach 1 h beträgt zwischen 7 und 25 g/g. Weiterhin wurde das Rückhaltevermögen (Retention) der Vliesstoffe nach 1h in Prüflösung A bestimmt. Diese beträgt zwischen 80-100%. Zudem wird der Schrumpf der verfestigten Vliesstoffe in Prüflösung A nach 1h in Prüflösung A bestimmt. Der Schrumpf der Polyvinylalkohol-Vliesstoffe beträgt abhängig von der Temperdauer und damit dem Vernetzungsgrad der Vliesstoffe zwischen 1 und 45%. Der Schrumpf der Mischvliesstoffe ist somit deutlich niedriger im Vergleich zu Polyvinylalkohol-Vliesstoffen ohne Zumischung der Polyvinylalkohol-Fasern, die ab einer Temperatur von 70°C wasserlöslich sind. Mit steigendem Anteil an dieser Fasern im Vliesstoff erniedrigt sich der Schrumpf.

Tabelle 4: Beispiel für einen nadelverfestigten Vliesstoff aus wasserlöslichen Polyvinylalkohol-Fasern mit Zumischung von Polyvinylalkohol-Fasern, die ab einer Temperatur von 70°C wasserlöslich sind, der getempert wurde

| Parameter | Beschreibung / Ergebnis |
|---|---|
| Polyvinylalkohol-Fasern | 2,2 dtex, 51 mm |
| Temperatur, bei der die Polyvinylalkohol-Fasern wasserlöslich sind | Unterhalb von 25°C |
| Anteil an Polyvinylalkohol-Fasern [%] | 50 bis 100 |
| Polyvinylalkohol-Fasern, die ab einer Temperatur von 70°C wasserlöslich sind | 1,7 dtex, 38 mm |

(fortgesetzt)

| Parameter | Beschreibung / Ergebnis |
|---|---|
| Anteil an Polyvinylalkohol-Fasern, die ab einer Temperatur von 70°C wasserlöslich sind [%] | 0 bis 50 |
| Temperdauer bei 150°C [min] | 150-300 |
| Verfestigungsart | Mechanisch durch Vernadelung |
| Einstichdichte [#/cm$^2$] | 100-170 |
| Flächengewicht [g/m$^2$] | 150-210 |
| Dicke [mm] | 0,8-3,0 |
| Relative Absorptionskapazität [g/g] nach 1 min in Prüflösung A | 7,0-25,0 |
| Relative Absorptionskapazität [g/g] nach 1h in Prüflösung A | 7,0-25,0 |
| Rückhaltevermögen (Retention) [%] nach 1h in Prüflösung A | 80-100 |
| Löslicher Anteil nach 1h in Prüflösung A [%] | 0-30 |
| Schrumpf [%] | 1 bis 45 % |
| Höchstzugkraft im hydrogeliertem Zustand [N/2cm]; längs | 4-30 |
| Höchstzugkraftdehnung im hydrogeliertem Zustand [%]; längs | 100-400 |
| Höchstzugkraft im hydrogeliertem Zustand [N/2cm]; quer | 4-30 |
| Höchstzugkraftdehnung im hydrogeliertem Zustand [%]; quer | 100-400 |

**Beispiel 6: Plasmabehandlung der getemperten Vliesstoffe zur Hydrophilierung**

Es wird ein nadelverfestigter Vliesstoff aus wasserlöslichen Polyvinylalkohol-Stapelfasern hergestellt. Die Polyvinylalkohol-Fasern sind bei einer Temperatur unterhalb von 25°C wasserlöslich und besitzen einen Fasertiter von 2,2 dtex bei einer Stapelfaserlänge 51 mm. Die Polyvinylalkohol-Fasern werden durch eine Krempel zu einem Vlies gelegt und anschließend durch Vernadelung mit einer Einstichdichte von 100-170 Einstichen pro Quadratcentimeter vernadelt. Die nadelverfestigten Polyvinylalkohol-Vliesstoffe werden bei einer Temperatur von 150°C getempert, um eine Stabilisierung des Polyvinylalkohols zu erzielen. Die Vliesstoffe werden dabei in einem handelsüblichen Labortrockenschrank mit Umluft über einen Zeitraum von 2,5 bis 5h getempert. Nach dem Tempern werden die Vliesstoffe im Vakuum mit Plasma unter Zuleitung von Sauerstoff behandelt, um die Hydrophilie der Vliesstoffe zu erhöhen. Alternativ wurden die Vliesstoffe nach dem Tempern mit einem Plasma unter Zuleitung von Sauerstoff und Acrylsäure behandelt. Bei beiden Plasma-Behandlungen wurde die Netzzeit von 2 min auf 1 s bis 10 s reduziert und damit die Hydrophilie der Vliesstoffe signifikant erhöht.

**Beispiel 7: Applikation von Netzmitteln auf den getemperten Vliesstoff zur Erhöhung der Hydrophilie**

Es wird ein nadelverfestigter Vliesstoff aus wasserlöslichen Polyvinylalkohol-Stapelfasern hergestellt. Die Polyvinylalkohol-Fasern sind bei einer Temperatur unterhalb von 25°C wasserlöslich und besitzen einen Fasertiter von 2,2 dtex bei einer Stapelfaserlänge 51 mm. Die Polyvinylalkohol-Fasern werden durch eine Krempel zu einem Vlies gelegt und anschließend durch Vernadelung mit einer Einstichdichte von 100-170 Einstichen pro Quadratcentimeter vernadelt. Die nadelverfestigten Polyvinylalkohol-Vliesstoffe werden bei einer Temperatur von 150°C getempert, um eine Stabilisierung des Polyivnylalkohols zu erzielen. Die Vliesstoffe werden dabei in einem handelsüblichen Labortrockenschrank mit Umluft über einen Zeitraum von 2,5 bis 5h getempert. Nach dem Tempern werden die Vliesstoffe mit einer wässrigen Lösung besprüht, die ein Netzmittel oder einen Filmbildner enthält, welche die Hydrophilie des Vliesstoffes erhöhen. Dazu werden wässrige Lösungen mit einer Konzentration von 5 bis 20% aus dem Netzmittel, Tenside oder Filmbildner hergestellt und diese mit Hilfe einer Druckluft-Sprühpistole auf den Vliesstoff gesprüht. Als Netzmittel, Tenside oder Filmbildner wurden folgende Substanzen verwendet: Tween 20, Conolan PG, Lertisan HD30, Lubricit 1136, Lubricit 1970, Polyethylenglykol mit einem Molekulargewicht von 400 g/mol.
Durch die Besprühung der Vliesstoffe wurde die Netzzeit von 2 min auf 1 s bis 10s reduziert und damit die Hydrophilie der Vliesstoffe signifikant erhöht..

**Beipiel 8: Antimikrobielle Ausstattung der Vliesstoffe aus ethanolischer Lösung**

Es wird ein nadelverfestigter Vliesstoff aus wasserlöslichen Polyvinylalkohol-Stapelfasern hergestellt. Die Polyvinylalkohol-Fasern sind bei einer Temperatur unterhalb von 25°C wasserlöslich und besitzen einen Fasertiter von 2,2 dtex bei einer Stapelfaserlänge 51 mm. Die Polyvinylalkohol-Fasern werden durch eine Krempel zu einem Vlies gelegt und anschließend durch Vernadelung mit einer Einstichdichte von 100-170 Einstichen pro Quadratcentimeter vernadelt. Die nadelverfestigten Polyvinylalkohol-Vliesstoffe werden bei einer Temperatur von 150°C getempert, um eine Stabilisierung des Polyvinylalkohols zu erzielen. Die Vliesstoffe werden dabei in einem handelsüblichen Labortrockenschrank mit Umluft über einen Zeitraum von 2,5 bis 5h getempert. Nach dem Tempern werden die Vliesstoffe durch Tränken in ethanolischer Polyhexanid-Lösung mit Polyhexanid ausgestattet. Dafür wird eine 0,4%ige Lösung von Polyhexanid in Ethanol (absolut) hergestellt. Für die Ausrüstung wird z.B. eine Beschichtungsanlage der Fa. Coatema (Basecoater oder Smartcoater) mit einem Foulard verwendet. Die Polyhexanid-Lösung wird in ein Foulardbecken gegeben. Der getemperte Vliesstoff läuft durch den Foulard und wird dabei mit der Polyhexanid-Lösung getränkt. Danach wird die Lösung durch Walzendruck in den Vliesstoff gepresst. Anschließend läuft der Vliesstoff durch einen Trockner mit einer Temperatur von 70°C, um den Vliesstoff zu trocken. Der Vliesstoff hat nach der Ausrüstung mit der Polyhexanid-Lösung einen Gehalt von 1 bis 5 g/m$^2$ Polyhexanid.

Tabelle 5: Ausrüstung eines getemperten Vliesstoffes mit Polyhexanid mit einem Foulard (Smarcoater, Fa. Coatema)

| | |
|---|---|
| Polyhexanid-Konzentration in Ethanol [Gew.-%] | 0,4 |
| Geschwindigkeit Vliesstoff: | 0,1m/min |
| Trocknerlänge | 3 m |
| Temperatur Trockner [°C] | 70°C |
| Polyhexanid-Gehalt im Vliesstoff | 3,05 /m$^2$ |

**Beispiel 9: Antimikrobielle Ausstattung der Vliesstoffe aus wässriger Lösung**

Es wird ein nadelverfestigter Vliesstoff aus wasserlöslichen Polyvinylalkohol-Stapelfasern hergestellt. Die Polyvinylalkohol-Fasern sind bei einer Temperatur unterhalb von 25°C wasserlöslich und besitzen einen Fasertiter von 2,2 dtex bei einer Stapelfaserlänge 51 mm. Die Polyvinylalkohol-Fasern werden durch eine Krempel zu einem Vlies gelegt und anschließend durch Vernadelung mit einer Einstichdichte von 100-170 Einstichen pro Quadratcentimeter vernadelt. Die nadelverfestigten Polyvinylalkohol-Vliesstoffe werden bei einer Temperatur von 150°C getempert, um eine Stabilisierung des Polyivnylalkohols zu erzielen. Die Vliesstoffe werden dabei in einem handelsüblichen Labortrockenschrank mit Umluft über einen Zeitraum von 2,5 bis 5h getempert. Nach dem Tempern werden die Vliesstoffe durch Tränken in wässriger Polyhexanid-Lösung mit Polyhexanid ausgestattet. Dafür wird eine 20%ige Lösung von Polyhexanid in destilliertem Wasser hergestellt. Für die Ausrüstung wird z.B. eine Beschichtungsanlage der Fa. Coatema (Basecoater oder Smartcoater) mit einem Kisscoater verwendet. Der Kisscoater läuft durch ein Bad, welches mit der 20%igen Polyhexanid-Lösung gefüllt ist, nimmt einen Teil der Lösung mit und beschichtet damit den Vliesstoff, der über den Kisscoater entlang geführt wird. Anschließend läuft der Vliesstoff durch einen Trockner mit einer Temperatur von 70°C, um den Vliesstoff zu trocken. Der Vliesstoff hat nach der Ausrüstung mit der Polyhexanid-Lösung einen Gehalt von 1 bis 5 g/m$^2$ Polyhexanid

Tabelle 6: Ausrüstung eines getemperten Vliesstoffes mit Polyhexanid mit einem Kisscoater (Smartcoater, Fa. Coatema)

| | |
|---|---|
| Polyhexanid-Konzentration in Ethanol [Gew.-%] | 15 bis 20 |
| Geschwindigkeit Vliesstoff: | 1 m/min |
| Trocknerlänge | 3 m |
| Temperatur Trockner [°C] | 70°C |
| Geschwindigkeit Walze Kisscoater | 1 m/min |
| Polyhexanid-Gehalt im Vliesstoff | 1 bis 5 g/m$^2$ |

**Beispiel 10: Bestimmung der Thermodesorption**

Bei nichtgetemperten PVA-Fasern können mittels Thermodesorption Dimethylsulfoxid und Fettalkoholethoxylate, z.B. aus der Avivage, nachgewiesen werden. Nach dem Tempern werden die getemperten PVA-Fasern ebenfalls mittels Thermodesorption untersucht. Es können nach dem Tempern weder Dimethylsulfoxid noch Fettalkoholethoxylate detektiert werden, so dass der Gehalt dieser unter den entsprechenden Nachweisgrenzen liegt. Durch das Tempern können somit im Vliesstoff oder den Fasern enthaltene Verunreinigungen, wie z.B. Spinnhilfsmittel, Lösungsmittel oder Avivage, entfernt werden.

**Beispiel 11: XPS-Bestimmungen von getemperten und ungetemperten Vliesstoffen**

Es wird ein nadelverfestigter Vliesstoff aus wasserlöslichen Polyvinylalkohol-Stapelfasern hergestellt. Die Polyvinylalkohol-Fasern sind bei einer Temperatur unterhalb von 25°C wasserlöslich und besitzen einen Fasertiter von 2,2 dtex bei einer Stapelfaserlänge 51 mm. Die Polyvinylalkohol-Fasern werden durch eine Krempel zu einem Vlies gelegt und anschließend durch Vernadelung mit einer Einstichdichte von 100-170 Einstichen pro Quadratcentimeter vernadelt. Die nadelverfestigten Polyvinylalkohol-Vliesstoffe werden bei einer Temperatur von 150°C getempert, um eine Stabilisierung des Polyivnylalkohols zu erzielen. Es werden mittels XPS ungetemperte und getemperte Vliesstoffe (nach verschiedenen Temperzeiten bei 150°Cmittels XPS untersucht.

Tabelle 7: XPS Übersichtspekrum

| | Ungetemperter Vliesstoff | Vliesstoff nach einer Temperzeit von 2 bis 3 Stunden bei 150°C | Vliesstoff nach einer Temperzeit von 4 bis 5 Stunden bei 150°C |
|---|---|---|---|
| C 1s [atom-%] | 81,6 | 84,1 | 84,8 |
| O 1s [atom-%] | 17,8 | 15,9 | 15,2 |
| P 2p [atom-%] | 0,6 | --- | --- |

Im XPS-Übersichtsspektrum ist zu erkennen, dass die Intensität des O 1s abnimmt. Dies kann durch eine Abnahme von Restwasser (Restfeuchtigkeit) durch das Tempern verursacht werden.

Tabelle 8: Hochaufgelöstes C 1s-Spektrum

| Signal | Ungetemperter Vliesstoff | Vliesstoff nach einer Temperzeit von 2 bis 3 Stunden bei 150°C | Vliesstoff nach einer Temperzeit von 4 bis 5 Stunden bei 150°C |
|---|---|---|---|
| C-C, C-H [area-%] | 74,4 | 84,3 | 86,2 |
| C-OH [area-%] | 23,9 | 10,3 | 5,7 |
| C=O [area-%] | --- | 4,0 | 5,5 |
| O-C=O [area-%] | 1,6 | 1,5 | 2,7 |

Aus dem hochaufgelösten C1 s-Spektrum ist zu erkennen: Mit zunehmender Temperdauer des Vliesstoffes nimmt der Anteil von Hydroxylgruppen im Vliesstoff ab. Gleichzeitig steigt der Anteil an Carbonyl-Gruppen im Vliesstoff signifikant an. Auch der Gehalt an Carboxy-Gruppen steigt an. Vermutlich werden durch das Tempern auch C-O-C-Vernetzungen ausgebildet.Die XPS-Untersuchungen zeigen, dass die Vliesstoffe aus wasserlöslichen Polyvinylalkohol-Fasern durch das Tempern eine Änderung in der chemischen Struktur aufweisen und diese dadurch eine hohe Stabilität gegenüber wässrigen Lösungen aufweisen.

**Patentansprüche**

1. Hydrogelierend ausgebildete Fasergebilde mit einem Flächengewicht von 20 bis 600 g/m$^2$, zwei oder dreidimensional, hergestellt aus Fasern aus einem ersten Faserrohmaterial, wobei das erste Faserrohmaterial wasserlöslichen Polyvinylalkohol und/oder Polyvinylalkohol-Copolymer enthält, wobei die hydrogelierende Ausbildung der Fasern oder des Fasergebildes durch Tempern des Faserrohmaterials bei einer vorbestimmten Tempertemperatur, die größer als eine Glasübergangstemperatur und kleiner ist als eine Schmelztemperatur oder Zersetzungstemperatur des verwendeten ersten Faserrohmaterials, sowie eine vorbestimmte Temperdauer lang bewirkt wird, wobei das Faserrohmaterial durch das Tempern vernetzt wird und wobei die Fasern aus dem ersten Faserrohmaterial einen Fasertiter von 0,5 bis 12 dtex aufweisen, **dadurch gekennzeichnet dass** die Fasergebilde eine Aufnahmekapazität für 0,9%ige, wässrige Natriumchlorid-Lösung oder Prüflösung A gemäß DIN 13726-1 Punkt 3.2.2.3 von 4-30 g/g aufweisen.

2. Fasergebilde nach Anspruch 1,
   **dadurch gekennzeichnet dass** das erste Faserrohmaterial als Polymer-Blend ausgebildet ist und zumindest ein weiteres Polymer aus der folgenden Gruppe aufweisen kann:
   Polyolefine, Polyamide, Polyester, Polyacrylnitrile, Polyvinylchloride, Elastane, Polyvinylchloride, Polyester, Polylactide, Polyglykolide, Polyesteramide, Polycaprolactone, Polyhexamethylenterephthalate, Polyhydroxybutyrate, Polyhydroxyvalerate, Polyvinylamine, Polyvinylacetate, Polyethylenglycole, Polyethylenoxide, Polyvinylpyrrolidone, Polyurethane, Polyacrylate, Cellulose, Cellulosederivate, regenerierte Cellulosen, wie z.B. Viskosen, Celluloseether, wie z.B. Carboxymethylcellulosen, Methyl-, Ethylcellulosen, Hydroxymethylcellulosen, Hydroxyethylcellulosen, Hydroxyalkylmethylcellulosen, Hydroxypropylcellulosen, Celluloseester, wie z.B. Celluloseacetat, oxidierte Cellulosen, bakterielle Cellulosen, Cellulosecarbonate, Alginate, Chitosane, Gelatinen, Kollagene, Stärken, Hyaluronsäuren, Pektine oder Agar.

3. Fasergebilde nach einem der vorhergehenden Ansprüche,
   **dadurch gekennzeichnet dass** die Fasern oder Fasergebilde zusätzlich weitere Fasern aus zumindest einem zweiten Faserrohmaterial aufweisen, wobei das zweite Faserrohmaterial ausgewählt sein kann aus der Gruppe von folgenden nicht-gelierenden Faserrohmaterialien:
   Polyolefine, Cellulosen, Cellulosederivate, regenerierte Cellulosen wie Viskose, Polyamide, Polyacrylnitrile, Elastane, Polyvinylchloride, tierische Naturfasern, wie z.B. Seide, pflanzliche Naturfasern, wie z.B. Baumwolle, und/oder Polyester und/oder aus der Gruppe von folgenden gelierenden Faserroh materialien:
   Alginate, Celluloseether, wie z.B. Carboxymethylcellulosen, Methyl-, Ethylcellulosen, Hydroxymethylcellulosen, Hydroxyethylcellulosen, Hydroxyalkylmethylcellulosen, Hydroxypropylcellulosen, Celluloseester, wie z.B. Celluloseacetat, oxidierte Cellulosen, bakterielle Cellulosen, Cellulosecarbonate, Gelatinen, Kollagene, Stärken, Hyaluronsäuren, Pektine, Agar, Polyvinylamine, Polyvinylacetate, Polyethylenglycole, Polyethylenoxide, Polyvinylpyrrolidone, Polyurethane und/oder Polyacrylate.

4. Fasergebilde nach Anspruch 3,
   **dadurch gekennzeichnet, dass** die Fasern aus dem erstem Faserrohmaterial oder die weiteren Fasern als Bikomponentenfasern und/oder Mehrkomponentenfasern ausgebildet sind und/oder wobei das zweite Faserrohmaterial als Polymer-Blend ausgebildet ist.

5. Fasergebilde nach Anspruch 3 oder 4,
   **dadurch gekennzeichnet dass** der Anteil an weiteren Fasern 10-50 Gew.% beträgt.

6. Fasergebilde nach einem der vorhergehenden Ansprüche,
   **dadurch gekennzeichnet dass** die Fasern oder Fasergebilde eine Höchstzugkraft gemessen in Anlehnung an EN 29073-03, wie unter Ziffer 8 des Teils "Ausführung der Erfindung" beschrieben, im hydrogelierten Zustand von 0,3 N bis 50 N aufweisen.

7. Fasergebilde nach einem der vorhergehenden Ansprüche,
   **dadurch gekennzeichnet dass** die Fasern oder Fasergebilde eine Retention für Wasser und/oder wässrige Lösungen, insbesondere für 0,9%ige, wässrige Natriumchlorid-Lösung oder Prüflösung A gemäß DIN 13726-1 Punkt 3.2.2., von über 70 % aufweisen.

8. Fasergebilde nach einem der vorhergehenden Ansprüche,
   **dadurch gekennzeichnet dass** ein Schrumpf des jeweiligen Fasergebildes in Wasser und/oder wässrigen Lösungen, insbesondere in 0,9%ige, wässriger Natriumchlorid-Lösung oder Prüflösung A gemäß DIN 13726-1 Punkt 3.2.2.3 maximal 60 % beträgt.

9. Verfahren zur Herstellung von hydrogelierend ausgebildeten Fasergebilden gemäß einem oder mehreren der vorangehenden Ansprüche, in dem Fasern oder Fasergebilde aus einem ersten Faserrohmaterial umfassend wasserlöslichen Polyvinylalkohol und/oder wasserlösliches Polyvinylalkohol-Copolymer bei einer vorbestimmten Tempertemperatur, die größer ist als eine Glasübergangstemperatur und kleiner ist als eine Schmelztemperatur des verwendeten ersten Faserrohmaterials eine vorbestimmte Temperdauer lang getempert werden, so dass die Fasern vernetzt werden.

10. Verfahren nach Anspruch 9,
**dadurch gekennzeichnet dass** die vorbestimmte Temperdauer 10min bis 14h beträgt.

11. Verfahren nach einem der Ansprüche 9 oder 10,
**dadurch gekennzeichnet dass,** insbesondere vor oder nach dem Tempern, ein Verfestigungsverfahren zur Herstellung eines zwei oder dreidimensionalen Fasergebildes angewendet wird.

12. Verfahren nach einem der Ansprüche 9 bis 11,
**dadurch gekennzeichnet dass** weitere Fasern aus zumindest einem zweiten Faserrohmaterial zugemischt werden.

13. Verwendung von Fasergebilden nach einem der Ansprüche 1 bis 8 zur Herstellung von Materialien für medizinische Anwendungen, von Wundauflagen, Wundverbänden, Nahtmaterialien, Implantaten, Tissue Engineering Scaffolds, Arzneimitteln, Trägermaterialien, Dämmstoffen, Filtermaterialien, technische Absorberprodukten, Produkten für den Lebensmittelbereich, Hygieneprodukten, Kosmetikprodukten, Haushaltsprodukten, wobei als Hygieneprodukte Damenhygieneprodukte, Windeln und Inkontinenzprodukte umfasst sind, wobei als Haushaltsprodukte Reinigungsmaterialien umfasst sind.

14. Wundverbände oder Wundauflagen enthaltend Fasergebilde nach einem der Ansprüche 1 bis 8.

**Claims**

1. Fibrous structures configured to be hydrogelling, having a basis weight of 20 to 600 g/m$^2$, two- or three-dimensional, made from fibres from a first fibre raw material, wherein the first fibre raw material contains water-soluble polyvinyl alcohol and/or polyvinyl alcohol copolymer, wherein the hydrogelling configuration of the fibres or the fibrous structure is brought about by heating the fibre raw material at a predetermined anneal temperature which is greater than any glass transition temperature and smaller than any melting temperature or decomposition temperature on the part of the first fibre raw material used, and for a predetermined anneal time, wherein the fibre raw material is crosslinked by the heating and wherein the fibres from the first fibre raw material have a fibre linear density weight of 0.5 to 12 dtex, **characterized in that** the fibrous structures have an uptake capacity in respect of 0.9% aqueous sodium chloride solution or test solution A according to DIN 13726-1 Point 3.2.2.3, of 4-30 g/g.

2. Fibrous structures according to Claim 1, **characterized in that** the first fibre raw material is configured as a polymer blend and may include at least one further polymer from the following group: polyolefins, polyamides, polyesters, polyacrylonitriles, polyvinyl chlorides, elastanes, polyvinyl chlorides, polyesters, polylactides, polyglycolides, polyesteramides, polycaprolactones, polyhexamethylene terephthalates, polyhydroxybutyrates, polyhydroxyvalerates, polyvinylamines, polyvinyl acetates, polyethylene glycols, polyethylene oxides, polyvinylpyrrolidones, polyurethanes, polyacrylates, cellulose, cellulose derivatives, regenerated celluloses, e.g. viscoses, cellulose ethers, e.g. carboxymethylcelluloses, methylethylcelluloses, hydroxymethylcelluloses, hydroxyethylcelluloses, hydroxyalkylmethylcelluloses, hydroxypropylcelluloses, cellulose esters, e.g. cellulose acetate, oxidized celluloses, bacterial celluloses, cellulose carbonates, alginates, chitosans, gelatins, collagens, starches, hyaluronic acids, pectins or agar.

3. Fibrous structures according to either preceding claim, **characterized in that** the fibres or fibrous structures additionally include further fibres from at least one second fibre raw material, wherein the second fibre raw material may be selected from the group of the following non-gelling fibre raw materials:
polyolefins, celluloses, cellulose derivatives, regenerated celluloses such as viscose, polyamides, polyacrylonitriles, elastanes, polyvinyl chlorides, animal-type natural fibres, e.g. silk, vegetable-type natural fibres, e.g. cotton, and/or polyesters and/or from the group of the following gelling fibre raw materials:

alginates, cellulose ethers, e.g. carboxymethylcelluloses, methylethylcelluloses, hydroxymethylcelluloses, hydroxyethylcelluloses, hydroxyalkylmethylcelluloses, hydroxypropylcelluloses, cellulose esters, e.g. cellulose acetate, oxidized celluloses, bacterial celluloses, cellulose carbonates, gelatins, collagens, starches, hyaluronic acids, pectins, agar, polyvinylamines, polyvinyl acetates, polyethylene glycols, polyethylene oxides, polyvinylpyrrolidones, polyurethanes and/or polyacrylates.

4. Fibrous structures according to Claim 3, **characterized in that** the fibres from the first fibre raw material or the further fibres are configured as bicomponent fibres and/or polycomponent fibres and/or wherein the second fibre raw material is configured as a polymer blend.

5. Fibrous structures according to Claim 3 or 4, **characterized in that** the proportion of further fibres is 10-50 wt%.

6. Fibrous structures according to any preceding claim, **characterized in that** the fibres or fibrous structures have a maximum breaking force, measured according to EN 29070-03, as described under number 8 of the "Execution of the Invention" section, in the hydrogelled state of from 0.3 N to 50 N.

7. Fibrous structures according to any preceding claim, **characterized in that** the fibres or fibrous structures have a retention of water and/or aqueous solutions, in particular for 0.9% aqueous sodium chloride solution or test solution A according to DIN 13726-1 Point 3.2.2., of more than 70%.

8. Fibrous structures according to any preceding claim, **characterized in that** a shrinkage of the respective fibrous structure in water and/or aqueous solutions, in particular in 0.9% aqueous sodium chloride solution or test solution A according to DIN 13726-1 Point 3.2.2.3, is not more than 60%.

9. Process for production of fibrous structures configured to be hydrogelling, according to one or more than one preceding claim, in which the fibres or fibrous structures comprising a first fibre raw material comprising water-soluble polyvinyl alcohol and/or water-soluble polyvinyl alcohol copolymer is heated at a predetermined anneal temperature, which is greater than any glass transition temperature and smaller than any melting temperature on the part of the first fibre raw material used, and so the fibres are crosslinked.

10. Process according to Claim 9, **characterized in that** the predetermined anneal time is in the range from 10 min to 14 h.

11. Process according to either of Claims 9 and 10, **characterized in that** a consolidating process is applied to produce a two- or three-dimensional fibrous structure, in particular before or after said heating.

12. Process according to any of Claims 9 to 11, **characterized in that** further fibres from at least one second fibre raw material are admixed.

13. Use of fibrous structures according to any of Claims 1 to 8 in the manufacture of materials for medical applications, of wound contact layers, wound dressings, suture materials, implants, tissue engineering scaffolds, medicinal products, carrier materials, insulants, filter materials, technical absorber products, products for the food sector, hygiene products, cosmetic products, household products, wherein femcare products, diapers and incontinence products are comprehended as hygiene products and cleaning materials are comprehended as household products.

14. Wound dressings or wound contact layers containing fibrous structures according to any of Claims 1 to 8.

**Revendications**

1. Structures de fibres configurées sous forme hydrogélifiante, ayant une masse surfacique de 20 à 600 g/m$^2$, bi- ou tridimensionnelles, fabriquées de fibres d'une première matière première de fibres, la première matière première de fibres contenant de l'alcool polyvinylique soluble dans l'eau et/ou un copolymère d'alcool polyvinylique, la configuration hydrogélifiante des fibres ou de la structure de fibres étant effectuée par traitement thermique de la matière première de fibres à une température de traitement thermique prédéterminée qui est supérieure à une température de transition vitreuse et inférieure à une température de fusion ou température de décomposition de la première matière première de fibres utilisée, et pendant une durée de traitement thermique prédéterminée, la matière première de fibres étant réticulée par le traitement thermique et les fibres de la première matière première de fibres présentant une densité linéaire de fibres de 0,5 à 12 dtex, **caractérisées en ce que** les structures de fibres présentent une capacité d'absorption pour une solution aqueuse de chlorure de sodium à 0,9 % ou une solution d'essai A selon DIN 13726-1 point 3.2.2.3, de 4-30 g/g.

2. Structures de fibres selon la revendication 1, **caractérisées en ce que** la première matière première de fibres est configurée sous la forme d'un mélange de polymères et peut comprendre au moins un polymère supplémentaire du groupe suivant :
les polyoléfines, les polyamides, les polyesters, les polyacrylonitriles, les polychlorures de vinyle, les élastanes, les polychlorures de vinyle, les polyesters, les polylactides, les polyglycolides, les polyesteramides, les polycaprolactones, les polyhexaméthylène-téréphtalates, les polyhydroxybutyrates, les polyhydroxyvalérates, les polyvinylamines, les polyacétates de vinyle, les polyéthylène glycols, les polyoxydes d'éthylène, les polyvinylpyrrolidones, les polyuréthanes, les polyacrylates, la cellulose, les dérivés de cellulose, les celluloses régénérées, telles que p. ex. les viscoses, les éthers de cellulose, tels que p. ex. les carboxyméthylcelluloses, les méthyl-, éthylcelluloses, les hydroxyméthylcelluloses, les hydroxyéthylcelluloses, les hydroxyalkylméthylcelluloses, les hydroxypropylcelluloses, les esters de cellulose, tels que p. ex. l'acétate de cellulose, les celluloses oxydées, les celluloses bactériennes, les carbonates de cellulose, les alginates, les chitosans, les gélatines, les collagènes, les amidons, les acides hyaluroniques, les pectines ou l'agar-agar.

3. Structures de fibres selon l'une quelconque des revendications précédentes, **caractérisées en ce que** les fibres ou les structures de fibres comprennent en outre des fibres supplémentaires d'au moins une deuxième matière première de fibres, la deuxième matière première de fibres pouvant être choisie dans le groupe constitué par les matières premières de fibres non gélifiantes suivantes :

les polyoléfines, les celluloses, les dérivés de cellulose, les celluloses régénérées telles que la viscose, les polyamides, les polyacrylonitriles, les élastanes, les polychlorures de vinyle, les fibres naturelles animales, telles que p. ex. la soie, les fibres naturelles végétales, telles que p. ex. le coton, et/ou les polyesters, et/ou dans le groupe constitué par les matières premières de fibres gélifiantes suivantes :
les alginates, les éthers de cellulose, tels que p. ex. les carboxyméthylcelluloses, les méthyl-, éthylcelluloses, les hydroxyméthylcelluloses, les hydroxyéthylcelluloses, les hydroxyalkylméthylcelluloses, les hydroxypropylcelluloses, les esters de cellulose, tels que p. ex. l'acétate de cellulose, les celluloses oxydées, les celluloses bactériennes, les carbonates de cellulose, les gélatines, les collagènes, les amidons, les acides hyaluroniques, les pectines, l'agar-agar, les polyvinylamines, les polyacétates de vinyle, les polyéthylène glycols, les polyoxydes d'éthylène, les polyvinylpyrrolidones, les polyuréthanes et/ou les polyacrylates.

4. Structures de fibres selon la revendication 3, **caractérisées en ce que** les fibres de la première matière première de fibres ou les fibres supplémentaires sont configurées sous la forme de fibres bicomposantes et/ou de fibres multicomposantes et/ou dans lesquelles la deuxième matière première de fibres est configurée sous la forme d'un mélange de polymères.

5. Structures de fibres selon la revendication 3 ou 4, **caractérisées en ce que** la proportion des fibres supplémentaires est de 10 à 50 % en poids.

6. Structures de fibres selon l'une quelconque des revendications précédentes, **caractérisées en ce que** les fibres ou les structures de fibres présentent une force de traction maximale, mesurée conformément à EN 29073-03, comme décrit au point 8 de la partie "réalisation de l'invention", à l'état hydrogélifié, de 0,3 N à 50 N.

7. Structures de fibres selon l'une quelconque des revendications précédentes, **caractérisées en ce que** les fibres ou les structures de fibres présentent une rétention d'eau et/ou de solutions aqueuses, en particulier d'une solution de chlorure de sodium aqueux à 0,9 %, ou d'une solution d'essai A selon DIN 13726-1 point 3.2.2., de plus de 70 %.

8. Structures de fibres selon l'une quelconque des revendications précédentes, **caractérisées en ce qu'**un retrait de la structure de fibres respectives dans l'eau et/ou dans des solutions aqueuses, en particulier dans une solution de chlorure de sodium aqueux à 0,9 % ou une solution d'essai A selon DIN 13726-1 point 3.2.2.3, est de 60 % maximum.

9. Procédé de fabrication de structures de fibres configurées sous forme hydrogélifiante selon l'une quelconque ou plusieurs des revendications précédentes, dans lequel des fibres ou des structures de fibres constituées d'une première matière première de fibres contenant de l'alcool polyvinylique soluble dans l'eau et/ou un copolymère d'alcool polyvinylique soluble dans l'eau sont soumises à un traitement thermique pendant une durée de traitement thermique prédéterminée, à une température de traitement thermique prédéterminée supérieure à une température de transition vitreuse et inférieure à une température de fusion de la première matière première de fibres utilisée, de sorte que les fibres soient réticulées.

10. Procédé selon la revendication 9, **caractérisé en ce que** la durée de traitement thermique prédéterminée est de 10 minutes à 14 h.

11. Procédé selon l'une quelconque des revendications 9 ou 10, **caractérisé en ce que**, notamment avant ou après le traitement thermique, un procédé de solidification est appliqué pour la fabrication d'une structure de fibres bi- ou tridimensionnelle.

12. Procédé selon l'une quelconque des revendications 9 à 11, **caractérisé en ce que** des fibres supplémentaires en au moins une deuxième matière première de fibres sont incorporées.

13. Utilisation de structures de fibres selon l'une quelconque des revendications 1 à 8, pour la fabrication de matériaux pour applications médicales, de compresses, de pansements, de matériaux de suture, d'implants, de supports d'ingénierie tissulaire, de médicaments, de matériaux supports, de matériaux isolants, de matériaux de filtration, de produits absorbants techniques, de produits pour le domaine alimentaire, de produits d'hygiène, de produits cosmétiques, de produits ménagers, les produits d'hygiène comprenant les produits d'hygiène féminine, les couches et les produits pour l'incontinence, et les produits ménagers comprenant les matériaux de nettoyage.

14. Pansements ou compresses contenant des structures de fibres selon l'une quelconque des revendications 1 à 8.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0130407 A1
- WO 2005103097 A1
- WO 2009085679 A
- EP 0745708 A1
- WO 2012048768 A

**In der Beschreibung aufgeführte Nicht-Patentliteratur**

- *J Mater Sci,* 2010, vol. 45, 2456-2465
- **JIANQI F et al.** EUROPEAN POLYMER JOUR. PERGAMON PRESS LTD, 01. August 2002, vol. 38, 1653-1658
- **LEI LI et al.** NANOTECHNOLOGY. IOP, 01. Dezember 2005, vol. 16, 2852-2860
- **AHMET CAV et al.** Journal of Applied Polymer Science. Wiley, 15. September 2013, vol. 129, 3140-3149